# EUROPEAN PATENT APPLICATION

(11) **EP 4 299 588 A2**
(43) Date of publication of application: **03.01.2024**
(21) Application number: 23206818.9
(22) Date of filing: 10.09.2020
(51) Int. Cl.: C07K 16/24

(54) **TREATMENT METHODS FOR EYE DISORDERS**

(30) Priority: 10.09.2019 US 201962898405 P; 15.01.2020 US 202062961548 P
(62) Divisional of application: 20862560.8
(71) Applicant: University of Pittsburgh- Of the Commonwealth System of Higher Education, Pittsburgh PA 15260 (US)
(72) Inventor: Sinha, Debasish, Clarksville, Maryland, 21029 (US); Byrne, Leah, Pittsburgh, Pennsylvania, 15201 (US); Stepicheva, Nadezda Anatolyevna, Wilkinsburg, Pennsylvania, 15221 (US); Ghosh, Sayan, Pittsburgh, Pennsylvania, 15232 (US); Hose, Stacey, Clear Spring, Maryland, 21722 (US)
(74) Representative: Fish & Richardson P.C.

(57) **Abstract**

A method of inhibiting neutrophil activation in retinal pigment epithelial (RPE) cells comprising administering an inhibitor of interferon (IFN) λ to a subject in need thereof, as well as a method of restoring lysosomal function of RPE cells comprising administering a polypeptide comprising βA1-crystallin or a nucleic acid encoding the polypeptide to a subject in need thereof, are provided. A method of rejuvenating Vacuolar-type H⁺-ATPase (V-ATPase) activity in RPE cells by modulating assembly and disassembly of the V-ATPase in a subject in need thereof also is provided. Additionally, a method of treating diabetic retinopathy in a subject comprising administering a polypeptide comprising βA1-crystallin or a nucleic acid encoding the polypeptide to the subject is provided.

## Description

### CROSS-REFERENCE TO RELATED APPLICATIONS

This patent application claims the benefit of U.S. Provisional Patent Application No. 62/898,405, filed September 10, 2019, and U.S. Provisional Patent Application No. 62/961,548, filed January 15, 2020, which are incorporated by reference.

### SEQUENCE LISTING

Incorporated by reference in its entirety herein is a nucleotide/amino acid sequence listing submitted concurrently herewith.

### BACKGROUND OF THE INVENTION

Age-related macular degeneration (AMD) is the world's leading cause of blindness among the elderly. It is projected that the number of people with AMD worldwide will be 196 million in 2020, increasing to 288 million in 2040. More than 15 million Americans are affected by AMD, and the costs of treatment are in excess of $350 billion. The most recent data suggest that more than 3 million additional people in the United States will be diagnosed with the disease by 2020.

The vast majority of patients suffer from atrophic (dry) AMD. However, due to the relentless progression of the disease, within 10 years they will develop advanced disease. The burden of dry AMD is increasing as the "baby boomers" age. Despite this growing population of afflicted individuals, no definitive treatment (other than the AREDS II formulation for intermediate AMD) or prevention for dry AMD is currently available. The dry type of AMD affects approximately 80-90% of individuals with the disease. Its cause is unknown, and it usually progresses more slowly than the wet type.

There is currently anti-VEGF therapy available for patients suffering from wet AMD, such as AVASTIN^{™} (bevacizumab), LUCENTIS^{™}(ranibizumab injection), and EYLEA^{™} (aflibercept). However the treatment regime (requires injections every month) is very expensive, does not work long-term, and does not help all patients.

Due to the large number of patients with atrophic AMD, there is a desire for a new treatment that targets early disease at a stage before vision is lost.

### BRIEF SUMMARY OF THE INVENTION

The invention provides a method of inhibiting neutrophil activation in retinal pigment epithelial (RPE) cells comprising administering an inhibitor of interferon (IFN) λ to a subject in need thereof. The inhibitor can be a neutralizing anti-IFNλ antibody, an antisense oligonucleotide, a small interfering ribonucleic acid (siRNA), a small hairpin RNA (shRNA), a non-antibody binding polypeptide, or a small molecule chemical compound. The antibody can be an immunoglobulin, an antibody fragment (e.g., Fab, Fab', F(ab')2, F(ab'), F(ab), Fv, and scFv), and an antibody analogue (e.g., affibody, repebody, affilin, DARPin, tetranectin, microbody, peptide aptamer, and avimer). When the inhibitor is shRNA, the shRNA can target one or more of the nucleotide sequences consisting of the group consisting of SEQ ID NOs: 11-17. The shRNA can be comprised in a vector (e.g., AAV vector).

The invention also provides a method of restoring lysosomal function of RPE cells comprising administering a polypeptide comprising βA1-crystallin or a nucleic acid encoding the polypeptide to a subject in need thereof.

The invention further provides a method of rejuvenating Vacuolar-type H⁺-ATPase (V-ATPase) activity in RPE cells by modulating assembly and disassembly of the V-ATPase in a subject in need thereof.

The subject (e.g., human) can have age-related macular degeneration (AMD) or be at risk for developing AMD, wherein the AMD can be wet AMD or atrophic (dry) AMD, which may include geographic atrophy. The subject can have Stargardt's macular retinal degeneration or be at risk for developing Stargardt's macular retinal degeneration. The subject can have a neurodegenerative disease or be at risk for developing a neurodegenerative disease, such as Alzheimer's disease or Parkinson's disease. Alternatively or additionally, the subject can have diabetic retinopathy (DR) or be at risk for developing DR.

Additionally, the invention provides a method of treating diabetic retinopathy in a subject comprising administering a polypeptide comprising βA1-crystallin or a nucleic acid encoding the polypeptide to the subject.

### BRIEF DESCRIPTION OF THE SEVERAL VIEWS OF THE DRAWINGS

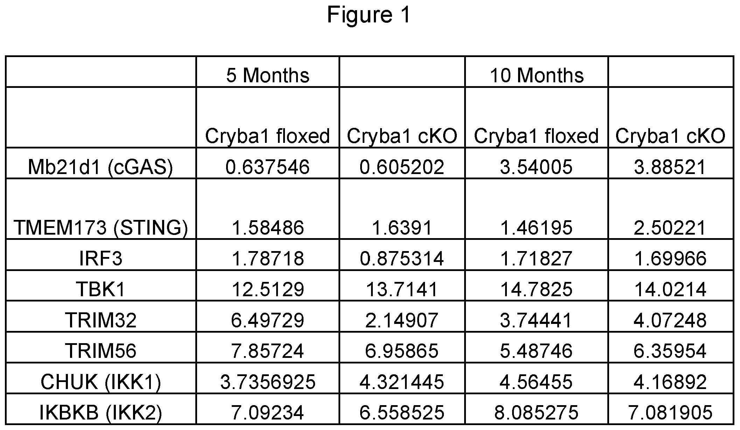

Figure 1 is a table of RNAseq analysis from RPE cells of 5 and 10 month old *Cryba1* floxed and *Cryba1* cKO mice showing expression of genes related to cGAS/STING.

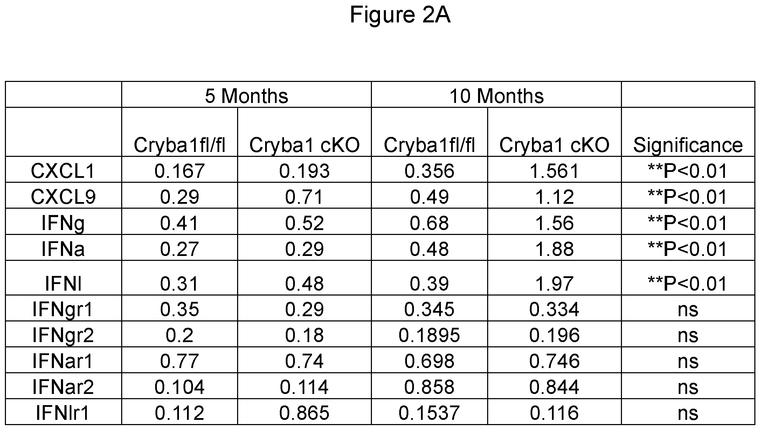

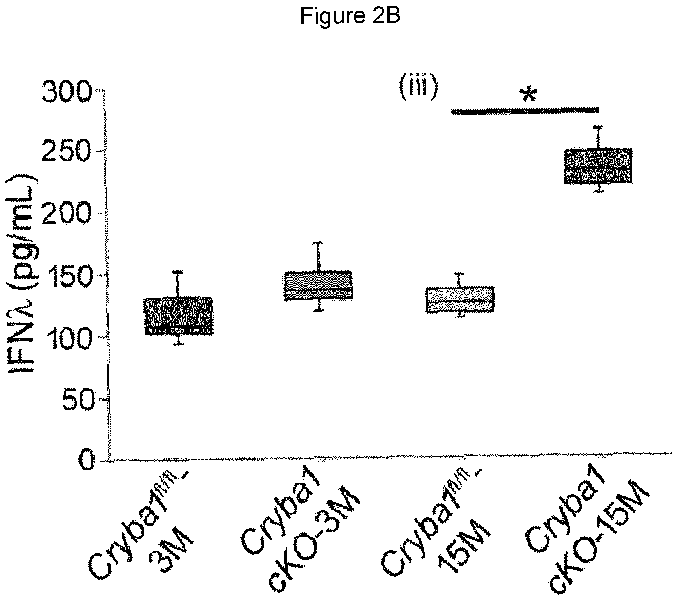

Figures 2A-B demonstrate activation of IFNλ in RPE cells form Crybal cKO mice with age. Figure 2A is a table of RNAseq analysis from RPE cells of 5 and 10 month old *Crybal* floxed and *Crybal* cKO mice. Significant increase in RNA levels of neutrophil regulating molecules like CXCL1, CXCL0, and IFN-family members, such as IFN Type I (IFNα, IFNβ), Type II (IFNγ), and Type III (IFNλ) in retina extracts from 10 month old *CrybaI* cKO mice compared to age-matched *Cryba*^{fl/fl} (control). No such changes were observed in 5 month old mice, no were there differences in expression of various IFN receptors. Represents Fragments per Kilobase of transcript per Million mapped reads (FPKM) for each gene and are represented as log10 (counts per million). Figure 2B is a graph showing the levels of IFNλ were increased in the RPE-choroid tissue homogenate of 15 month old *Crybal* cKO mice compared to age-matched *Crybal*^{fl/fl} controls as measured by ELISA. No changes were observed in 3 month old mice. n=6 *P<0.05 (one-way ANOVA and Tukey's post-hoc test).

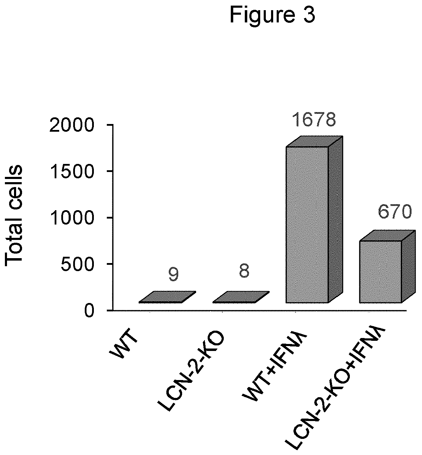

Figure 3 demonstrates that IFNλ triggers neutrophil homing into the retina of NOD-SCID mice. Counts extracted from all groups demonstrated an increase in neutrophil number (cell count) in the NOD-SCID mice injected (intravenous) with IFNλ-treated WT neturophils compared to untreated controls. Loss of LCN-2 in neutrophils (LCN-2^{-/-}) showed reduced infiltration even after IFNλ exposure. n=1. Scale bar, 500 µm.

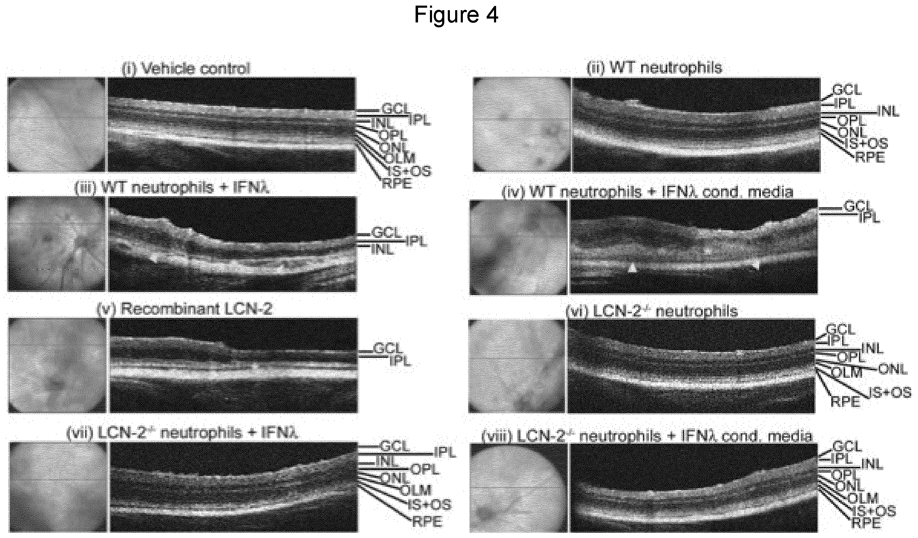

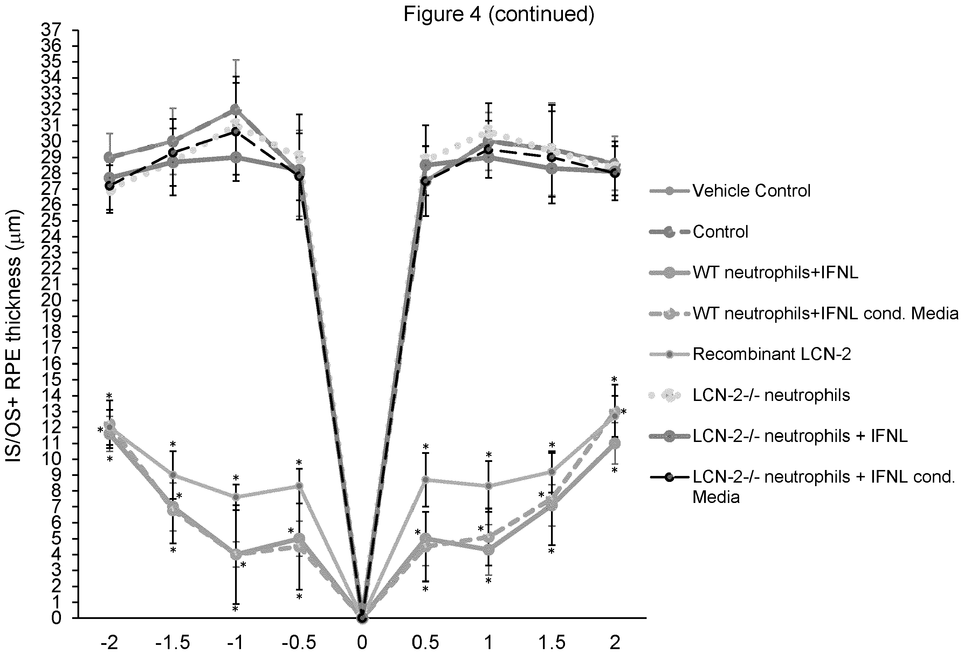

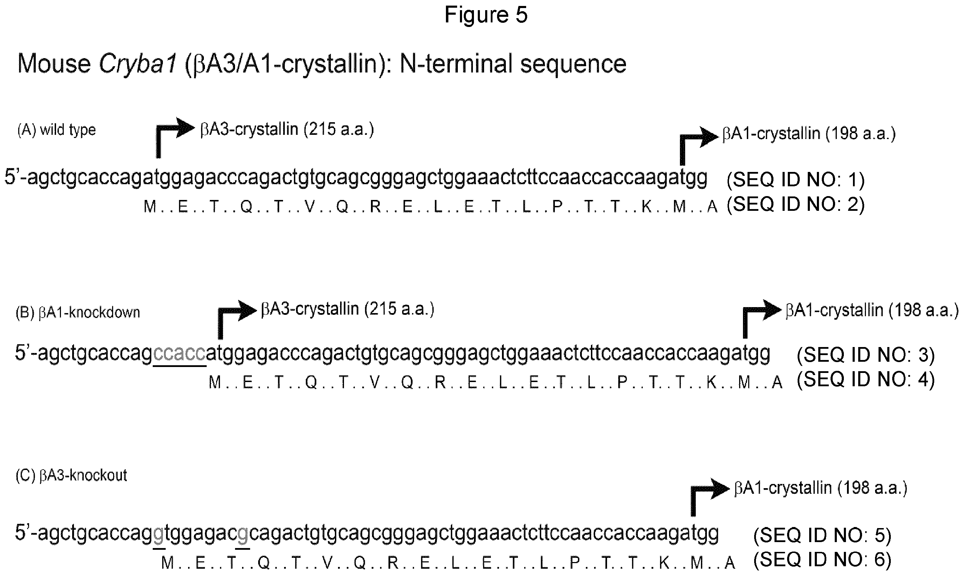

Figures 4(i)-(ix) demonstrate that IFNλ activated neutrophils cause retinal degeneration *in vivo.* Representative spectral-OCT images of retinas from NOD-SCID mice injected subretinally with (i) vehicle (HBSS) or (ii) WT neutrophils revealed normal retinal structure. In contrast, mice injected with WT neutrophils pre-treated with either (iii) recombinant IFNλ (200 U/mL), (iv) conditioned media from IFNλ overexpressing mouse RPE cells (1:1 diluted), or (v) recombinant LCN-2 (10 pg/mL) show apparent changes in the ONL and INL layers (asterisks), concomitant with severe loss of RPE and IS+OS layer (yellow arrow heads). These alterations were not observed in mice injected with (vi) untreated neutrophils from LCN-2^{-/-} mice or (vii-viii) IFNλ-exposed LCN-2^{-/-} neutrophils. Figure 5(ix) is a representative spider plot showing the thickness of the IS/OS+RPE layers using the OCT images among the experimental groups. n=10. **P*<0.05 (one-way ANOVA and Tukey's post-hoc test).
Figures 5A-C shows the mouse *Crybal* (βA3/A1-crystallin) N-terminal sequence in wild-type (A), βA1-knockdown (B), and βA3-knockout mice. The wild-type sequence of Crybal shows dual ATG start sites for βA3- and βA1-crystallin (A). βA1-knockdown mutant (B) shows insertion of CCACC (underlined) immediately before the first start site to strengthen the Kozak consensus sequence, thereby making the first start codon stronger. βA3-knockout mutant (C) showing A to G (underlined) change that inactivates the first start site. A silent mutation (ACC to ACG; change is underlined) also was introduced to prevent the binding and re-cutting of the sequence by gRNA after homology-directed repair.

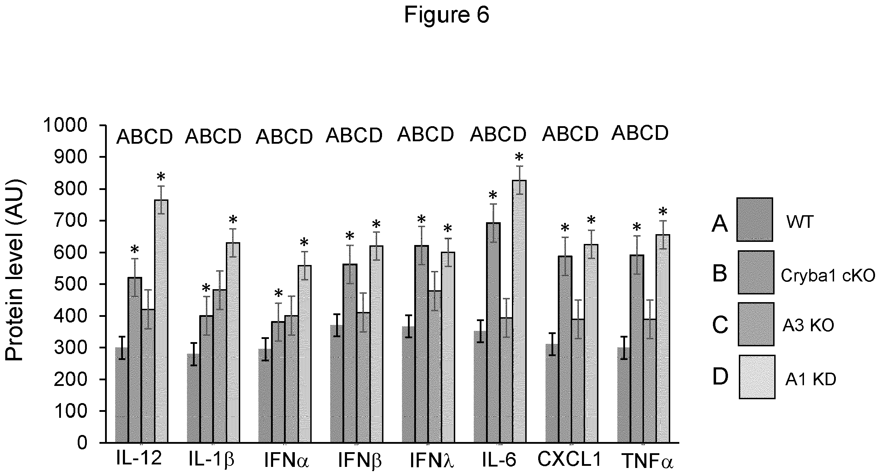

Figure 6 demonstrates that knockdown of βA1-crystallin causes inflammation in RPE cells. ELISA showing the protein levels of different cytokines form RPE culture supernatant in WT (A), Crybal cKO (B), βA3-knockout (C), and (D). *P<0.5.

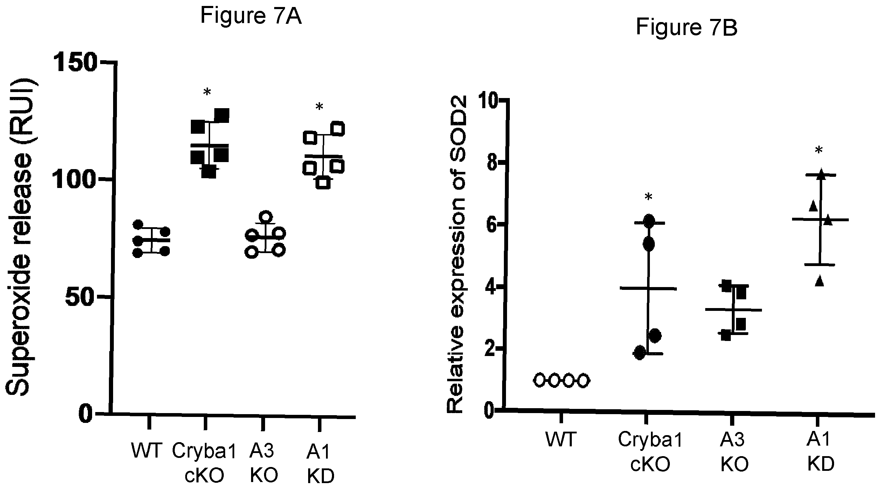

Figures 7A-B demonstrates that knockdown of βA1 crystallin causes oxidative damage in RPE cells. Figure 7A shows superoxide release from RPE cells of WT, *Cryba1* cKO, βA3-knockout (A3 KO), and βA1-knockdown (A1 KD) in culture. Figure 7B shows SOD2 expression by qPCR showing significant increase in *Cryba1* cKO and A1 KD cells relative to WT cells. **P*<0.05.

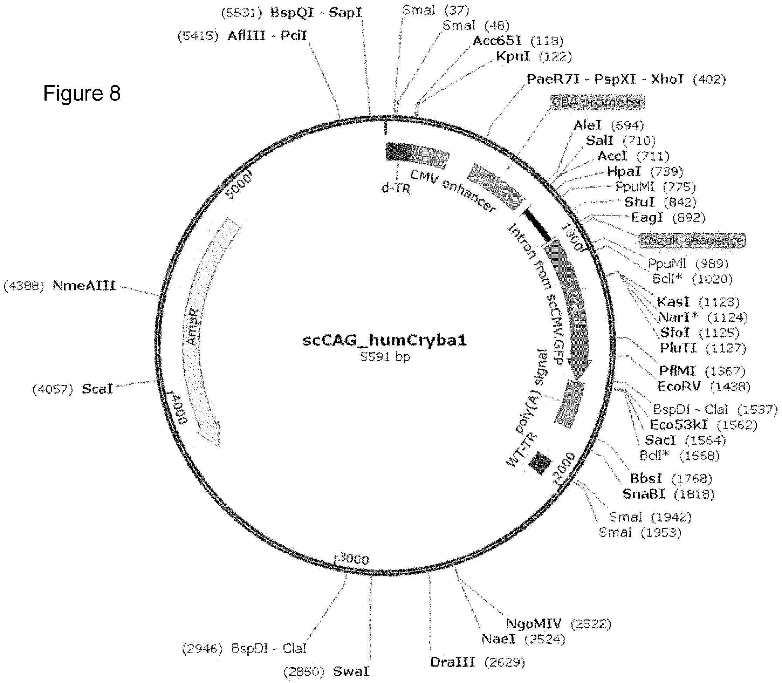

Figure 8 is a vector map of scCAG_humCryba1.

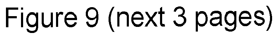

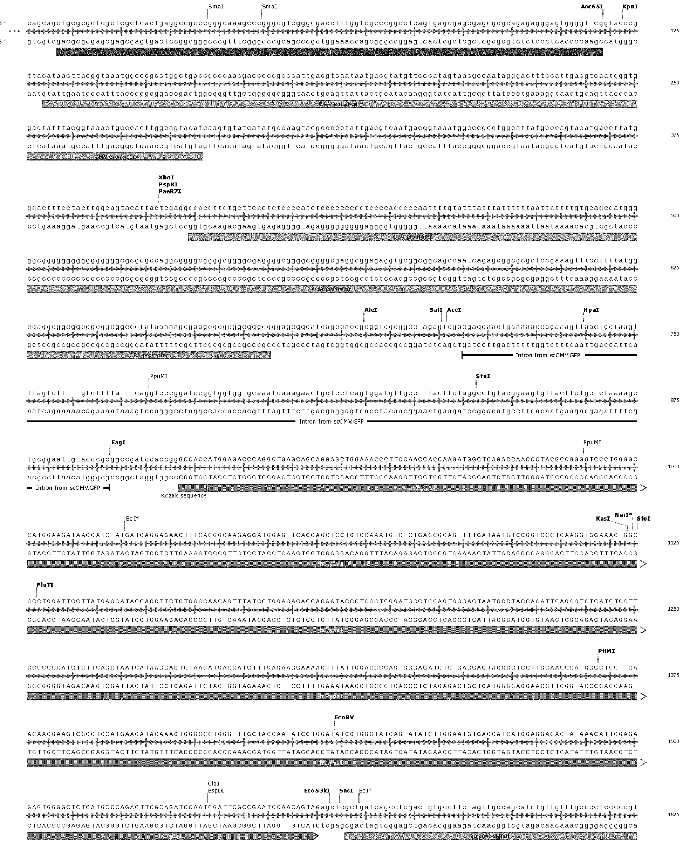

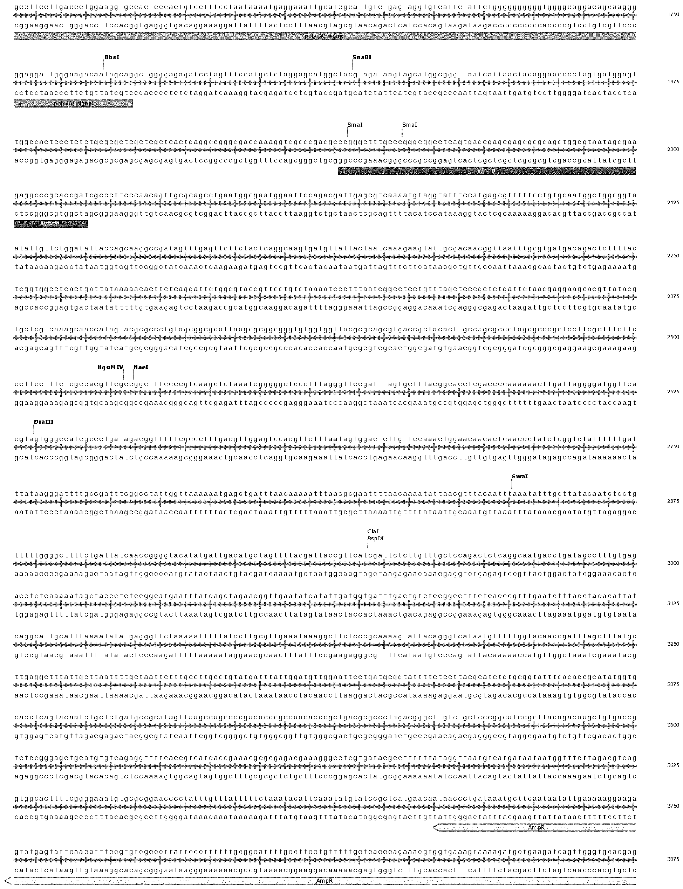

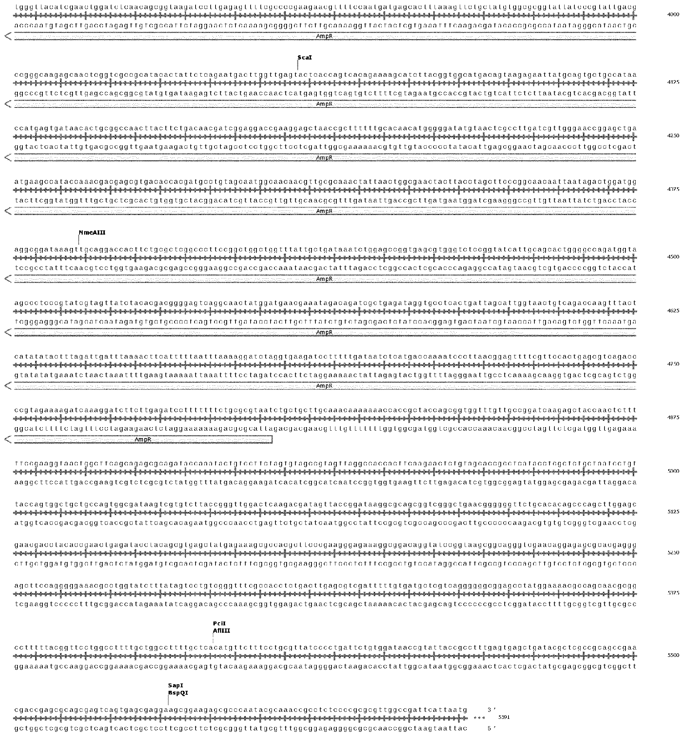

Figure 9 is the nucleotide sequence of scCAG_humCryba1 (SEQ ID NO: 18).

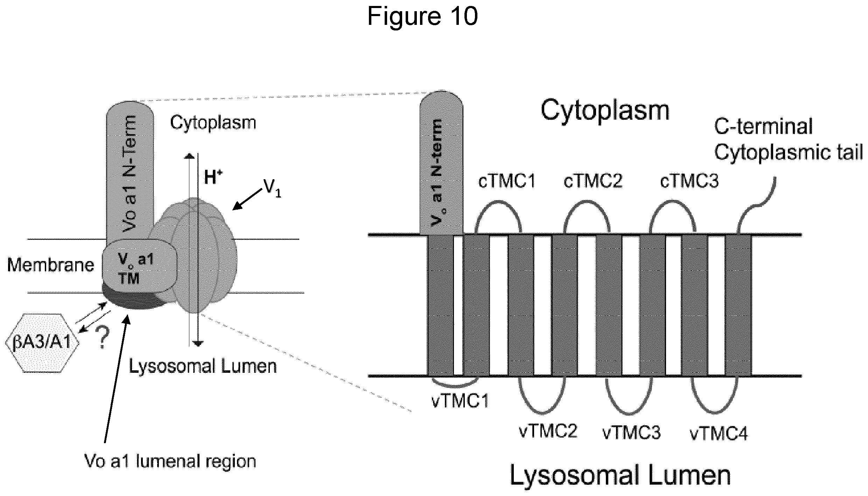

Figure 10 is a schematic demonstrating that βA3/A1-crystallin binds to V-ATPase in the lysosomal lumen. The left side shows the possible interaction between βA3/A1-crystallin and V-ATPase in the lysosomal lumen. In the schematic representation of the V-ATPase rotor (Vi) and stator (V₀) shows only the ATP6V₀A1 subunit for V₀. The ride side shows an enlarged schematic of the V0a1 subunit. ATP6V₀A1 N-term, cytoplasmic transmembrane connecting loops (cTMCs), and the C-terminal tail are distributed on the cytoplasmic side. The transmembrane helices are within the membrane (solid rectangles). The vacuolar transmembrane connecting loops (vTMCs) are on the lumenal side. Rat ATP6V₀A1/V₀-ATPase (Uniprot #P25286) residue boundaries: N-term (1-388), cTMC1 (427-441), cTMC2 (556-573), cTMC3 (660-725), C-tail (811-838), vTMC1(408-409), vTMC2(472-535), vTMC3(595-639), vTMC4(751-771). The human and rat sequences are very well conserved (Human ATP6V₀A1/V₀-ATPase is Uniprot # Q93050) with only minor changes in residue boundaries as compared to the rat: N-term (1-388), cTMC1 (427-441), cTMC2 (555-572), cTMC3 (659-724), C-tail (810-837), vTMC1(408-409), vTMC2(472-534), vTMC3(594-638), vTMC4(750-770).

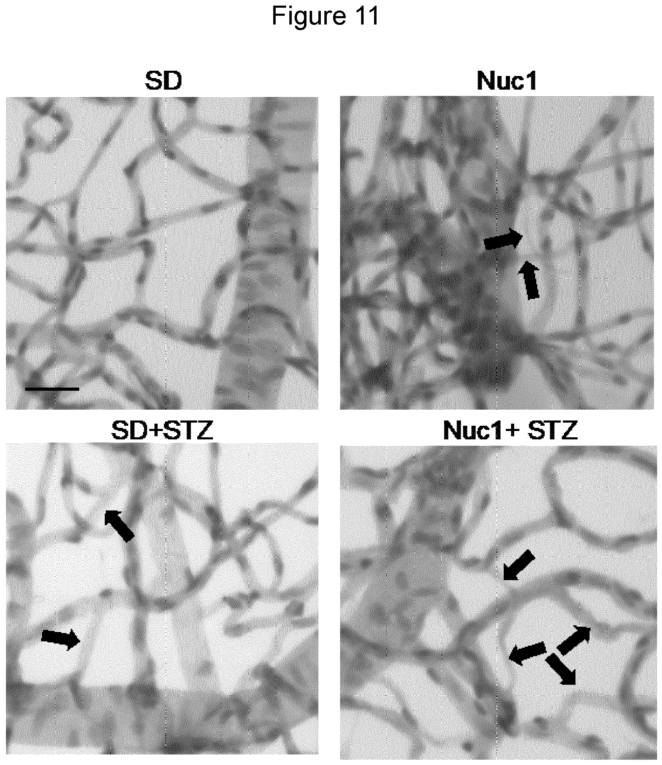

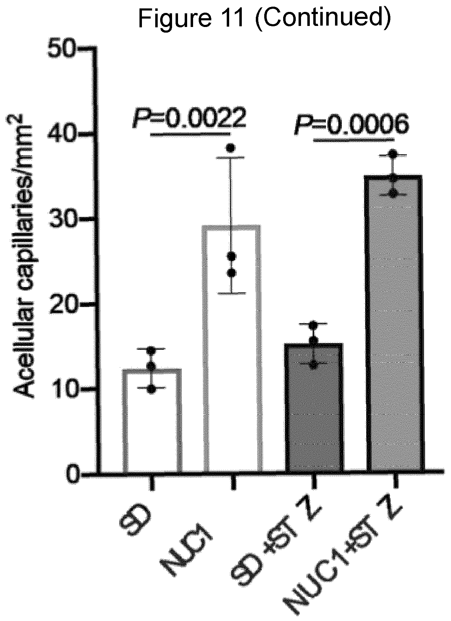

Figure 11 demonstrates the Nuc1 rat model is a diabetic retinopathy model. Arrows indicate degenerating vessels without any nuclei, which is a key marker for retinal vascular changes in diabetic retinopathy. As demonstrated in the images on the top left, degenerating vessels without nuclei were observed in Nuc1 rats but not Sprague-Dawley (SD) rats. Addition of the toxin streptozotocin (STZ) to the SD and Nuc1 rats also resulted in degenerating vessels without nuclei. The graph on the right demonstrates the number of acellular capillaries observed in the rats.

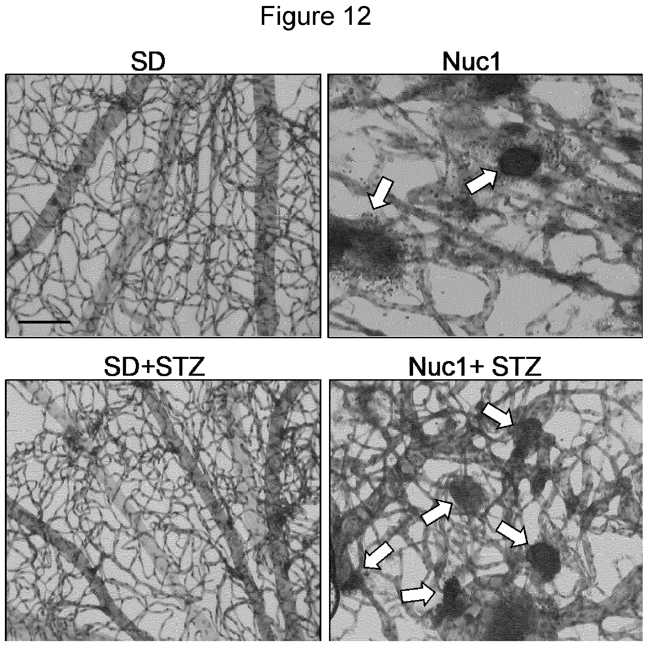

Figure 12 demonstrates the presence of microaneurysms in the retina of diabetic Nuc1 rats. Arrows indicate microaneurysms, which is a key marker for retinal vascular changes in diabetic retinopathy. Microaneurysms were not observed in SD mice even after administration of the toxin streptozotocin (STZ).

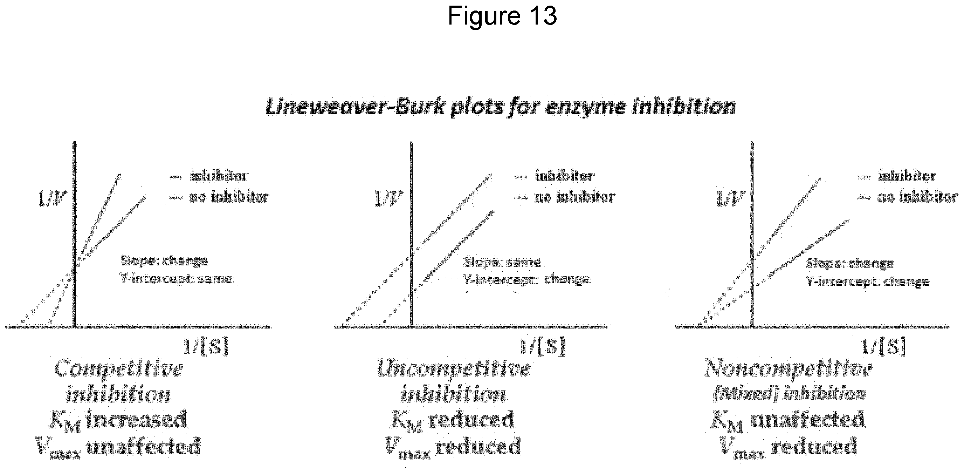

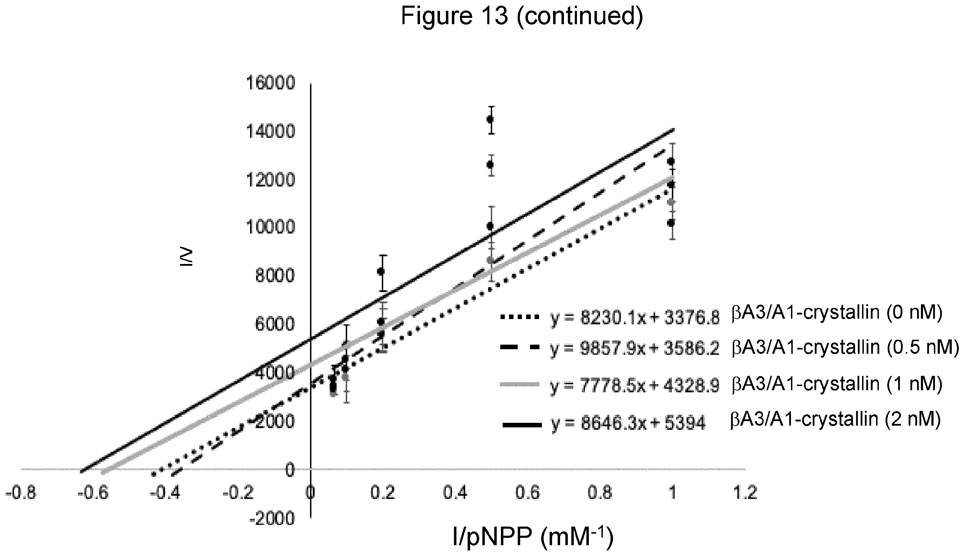

Figure 13 demonstrates that βA3/A1-crystallin is an uncompetitive inhibitor of PTP1B. Lineweaver Burk plots show that Crybal treatment decreases the Km and Vmax of PTP1B activity.

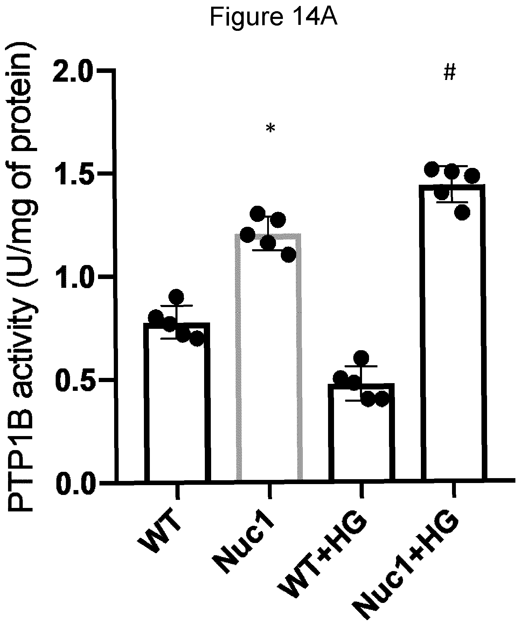

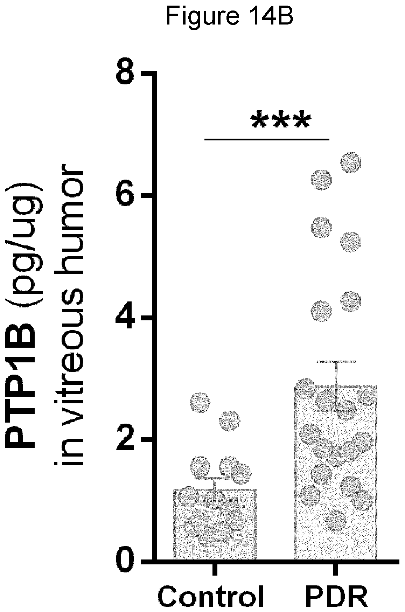

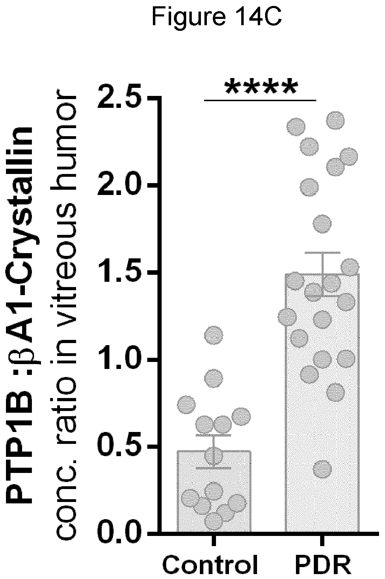

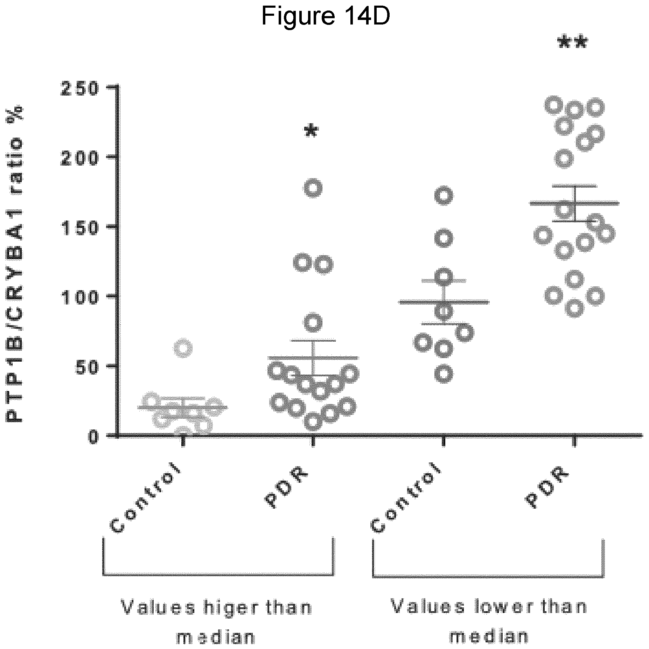

Figures 14A-14D demonstrate alterations in PTP1B activity and expression in Nuc1 rat model and human diabetic retinopathy patients. Fig. 14A shows the PTP1B activity (U/mg of protein) in wild-type and Nuc1 astrocytes in low and high glucose (HG) conditions. Fig. 14B demonstrates the elevated level of PTP1B (pg/µg) in vitreous humor of human DR patients as compared to control. Figs. 14C-14D demonstrate that samples from vitreous humor of human patients with progressive DR (PDR) showed lower levels of crystallin compared to PTP1B, but this was not observed in normal samples (control). *P<0.05, **P<0.01, ****P<0.0001; Mann Whitney Test.

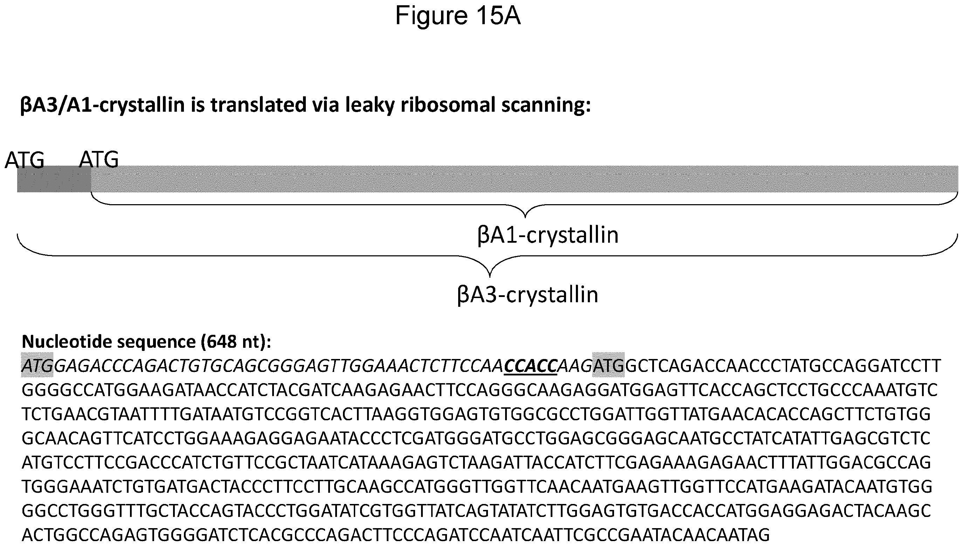

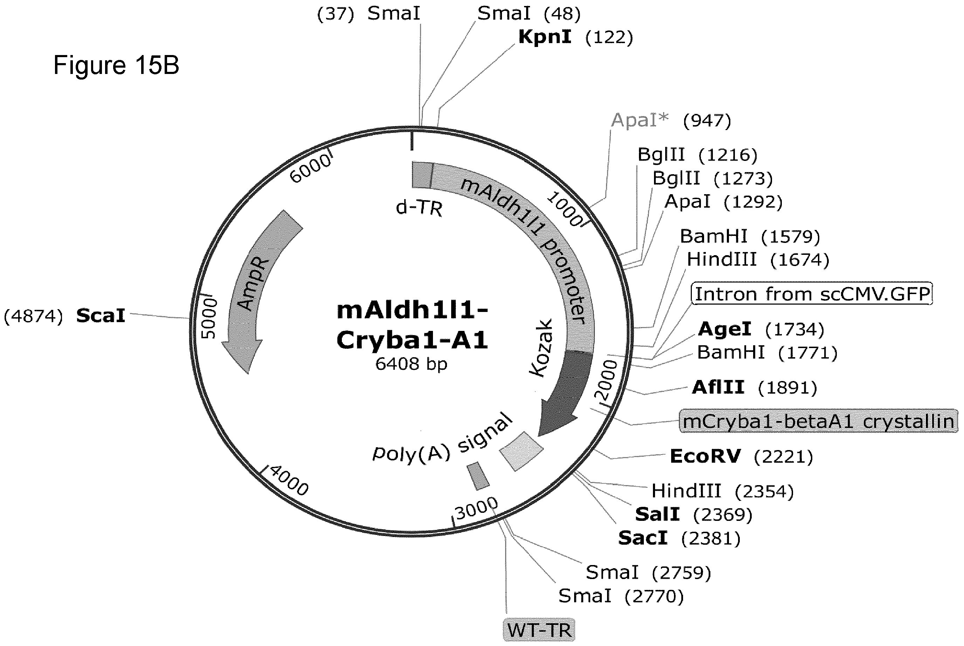

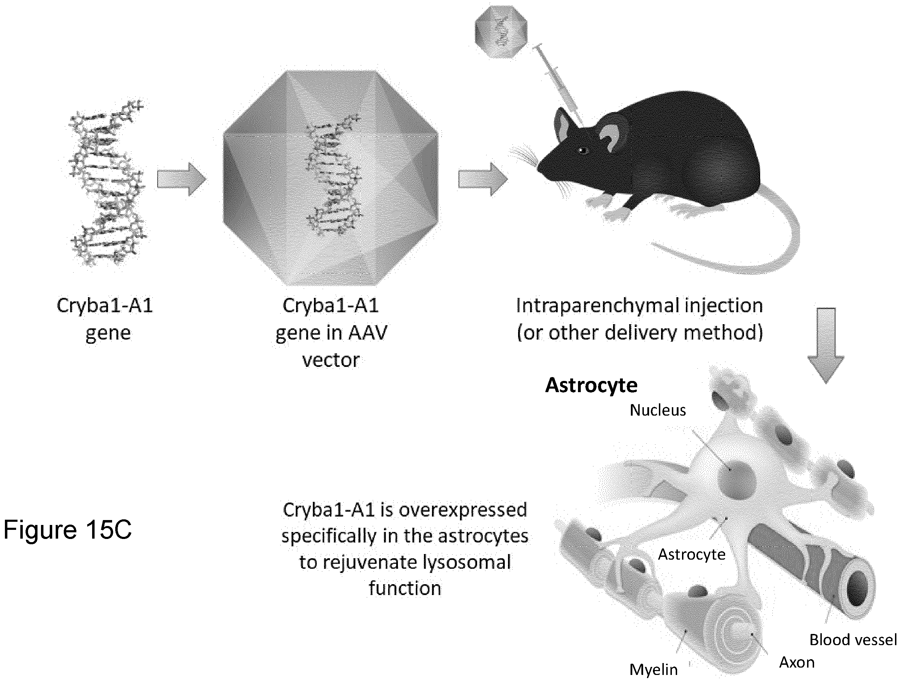

Figures 15A-15C depict a βA1-crystallin construct for gene therapy. The sequence corresponding to the rat βA1-crystallin (beginning at second ATG) (SEQ ID NO: 19) was cloned into the AAV2 transfer vector that contains rat Aldh1L1 promoter to ensure specific expression in astrocytes. The Crybal-A1 gene under the control of Aldh1L1 promoter was delivered by intravitreal injection to Nuc1 rats.

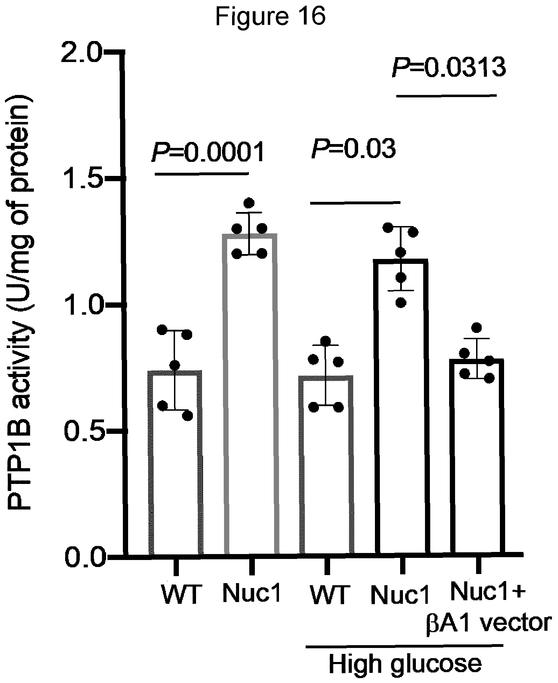

Figure 16 is a graph demonstrating that βA1-crystallin reduces abnormal PTP1B activity in Nuc1 astrocytes. The PTP1B activity (U/mg of protein) in wild-type and Nuc1 astrocytes in low and high glucose (HG) conditions is shown, wherein addition of a vector encoding βA1-crystallin reduces abnormal PTP1B activity in Nuc1 astrocytes.

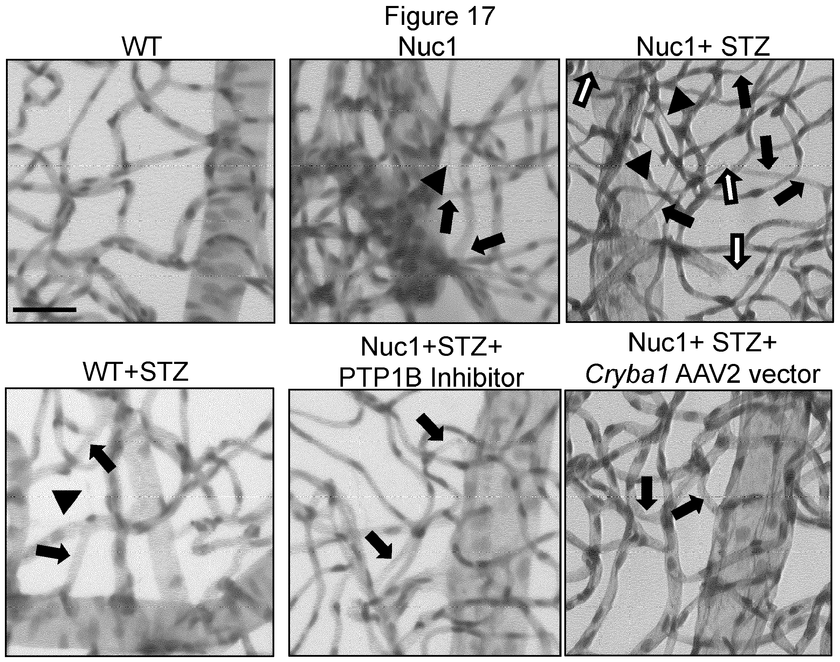

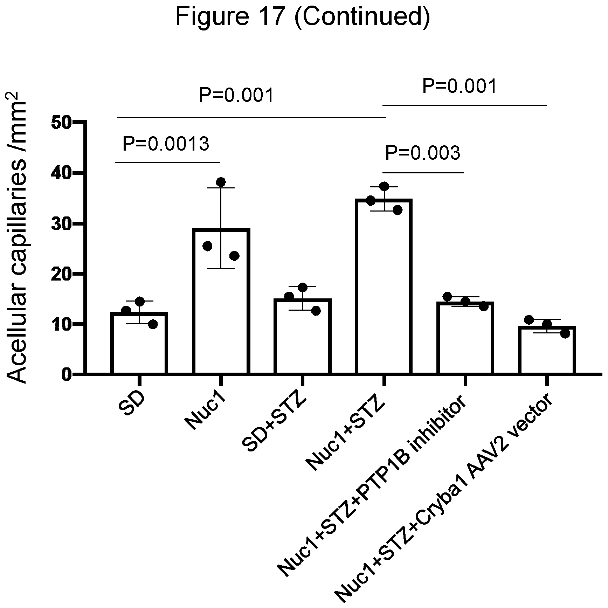

Figure 17 demonstrates that βA1-crystallin rescues the DR phenotype in Nuc1. In the images on the left, black arrows indicate acellular capillaries, black arrow heads indicate acellular capillaries with vascular thinning, and empty arrows indicate capillary loss. The graph on the right demonstrates the number of acellular capillaries observed in the rats. Scale bar: 100 mm.

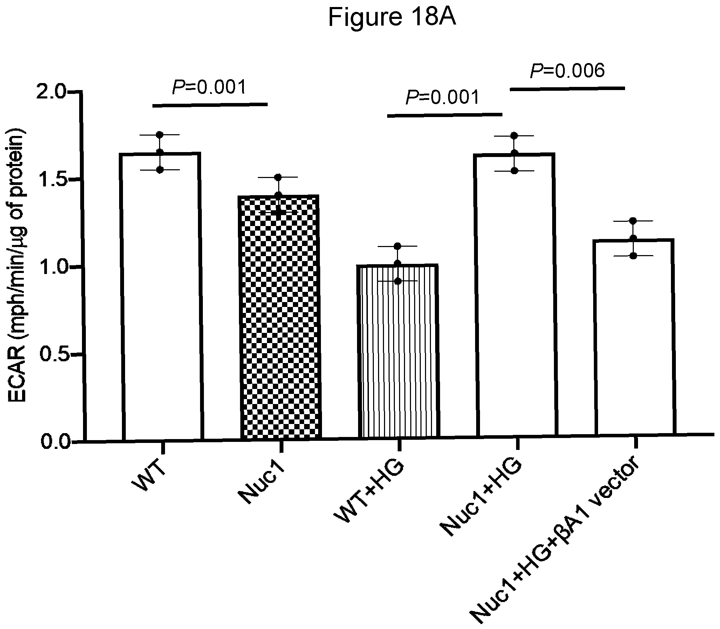

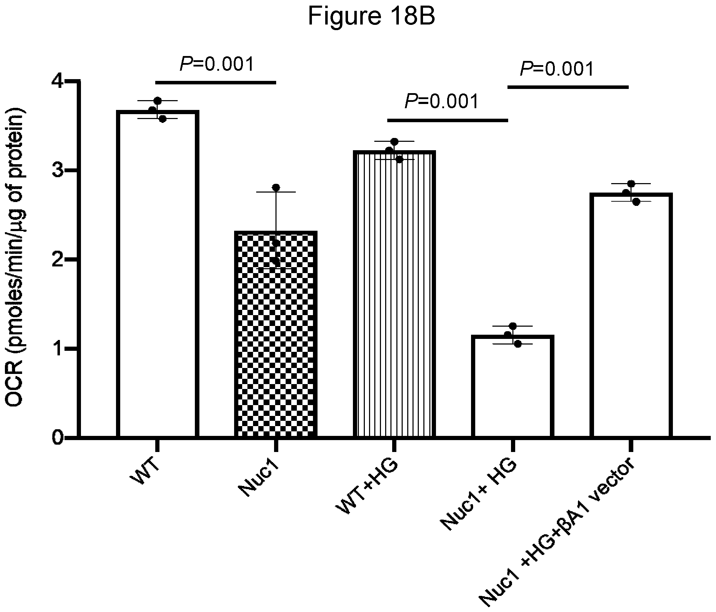

Figures 18A-18B demonstrate that βA1-crystallin rescues abnormal glucose metabolism in Nuc1 astrocytes. An increase in the extracellular acidification rate (ECAR; Fig. 18A) and a decrease in mitochondrial respiration (oxygen consumption rate or OCR; Fig. 18B) was observed in Nuc1 astrocytes treated with high glucose (HG) compared to HG-exposed WT cells. Overexpression of βA1-crystallin in Nuc1 + HG cells rescued both ECAR and OCR levels.

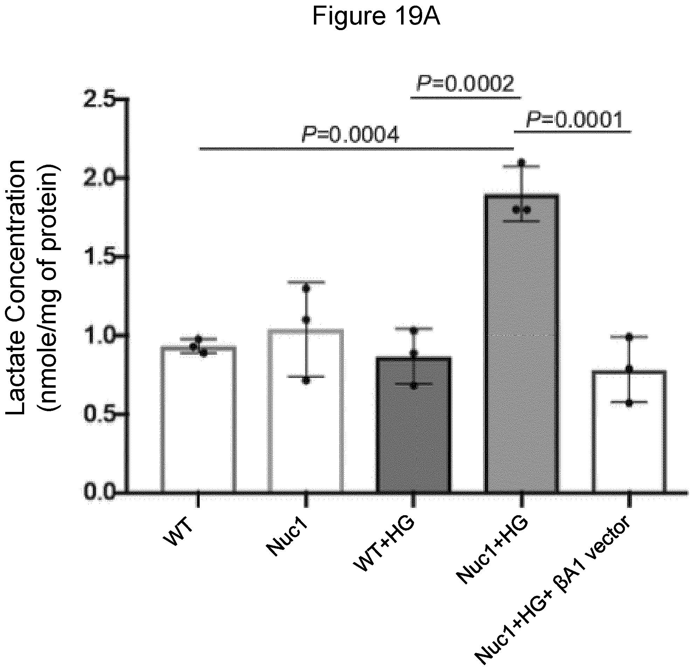

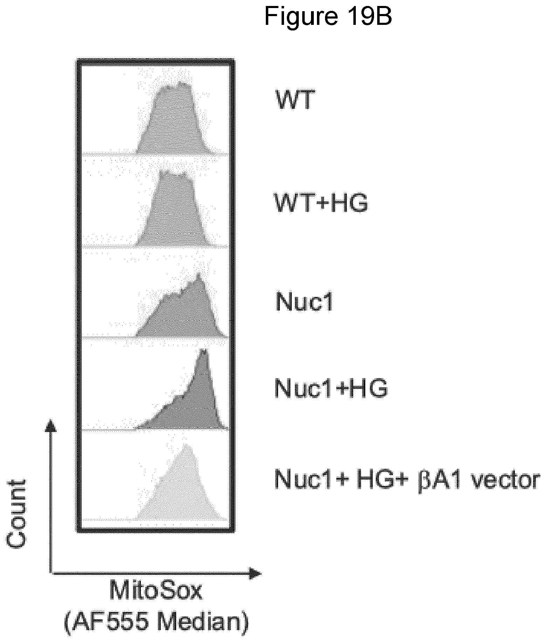

Figures 19A-19B demonstrate that βA1-crystallin reduces lactate production and mitochondrial oxidative stress in Nuc1 astrocytes under hyperglycemic conditions. Increased lactate levels and mitochondrial reactive oxygen species (ROS) are cardinal signs of metabolic alterations. An increase in lactate levels (Fig. 19A) and mitochondrial ROS (Fig. 19B) in HG-exposed Nuc1 astrocytes compared to control (WT cells) was reduced after βA1-crystallin overexpression.

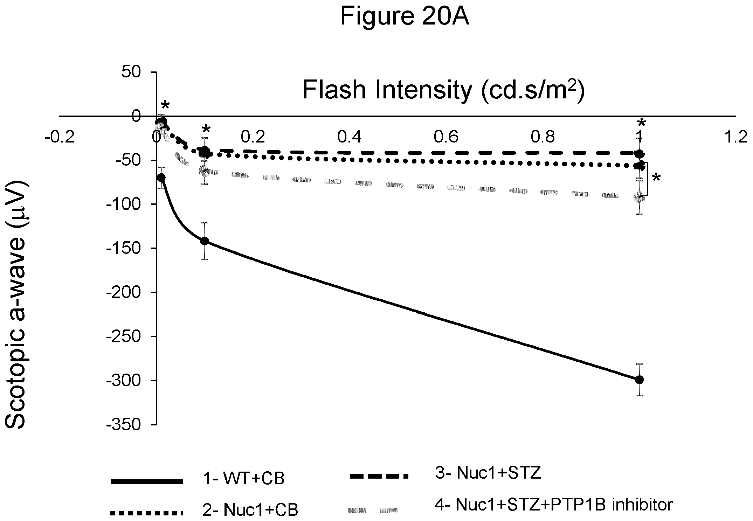

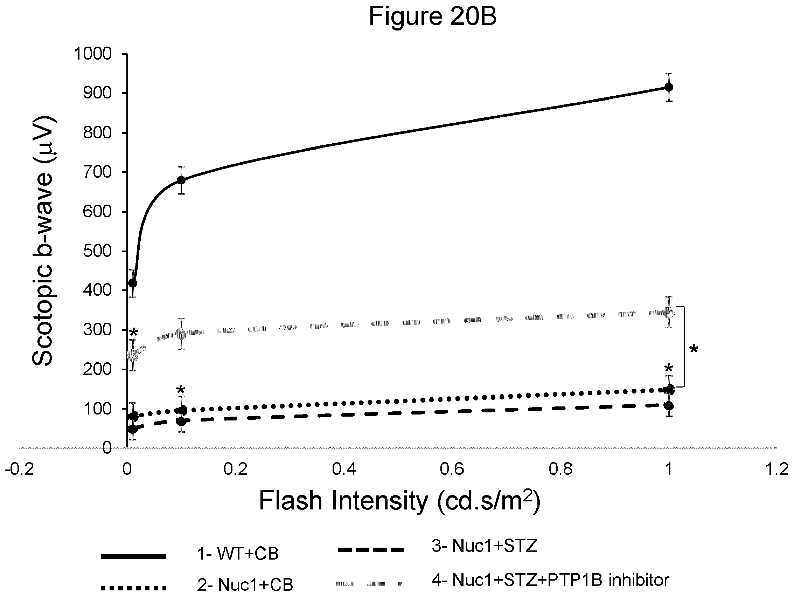

Figures 20A-20B demonstrate that PTP1B inhibitor (MSI-1436) rescues retinal function abnormality in diabetic Nuc1 rats. Similar results were observed by injecting βA1-crystallin vector intravitreally in diabetic Nuc1 astrocytes.

### DETAILED DESCRIPTION OF THE INVENTION

Age-related macular degeneration (AMD) is an expanding problem as longevity increases worldwide. While inflammation clearly contributes to vision loss in AMD, the mechanism remains controversial. The inventors discovered that neutrophils are important in this inflammatory process. In the retinas of both AMD patients and in a mouse model with an AMD-like phenotype, neutrophil infiltration was observed. Such infiltration was confirmed experimentally using ribbon-scanning confocal microscopy (RSCM) and IFNX-activated dye labeled normal neutrophils.

The inventors have developed a unique construct that overexpresses only βA1-crystallin of the leaky ribosomal protein βA3/A1-crystallin and packaged it in AAV2 with a hVMD2 promoter. The crystallin protein can rejuvenate the lysosomal function in RPE cells and rescue the AMD-like phenotype. This concept is significant beyond AMD, and can also be used for other disorders, such as diabetic retinopathy by targeting astrocytes.

In that respect, the invention provides a method of restoring lysosomal function of RPE cells comprising administering a polypeptide comprising, consisting essentially of, or consisting of βA1-crystallin or a nucleic acid encoding the polypeptide to a subject in need thereof.

While not wishing to be bound by any particular theory, it is believed that the administration of βA1-crystallin (i) reduces abnormal PTP1B activity, (ii) reduces abnormal glucose metabolism, (iii) reduces lactate production, (iv) reduces mitochondrial oxidative stress, and (v) rescues retinal function abnormality, thereby inhibiting, reducing, and/or treating diabetic retinopathy in affected subjects (see Example 9). Therefore, the invention provides related methods (e.g., methods of reducing or inhibiting PTP1B activity, glucose metabolism, lactate production, and/or mitochondrial oxidative stress) comprising administering a polypeptide comprising, consisting essentially of, or consisting of βA1-crystallin or a nucleic acid encoding the polypeptide to a subject in need thereof. In particular, the invention provides a method of treating diabetic retinopathy in a subject comprising administering a polypeptide comprising, consisting essentially of, or consisting of βA1-crystallin or a nucleic acid encoding the polypeptide to the subject.

The invention also provides a method of inhibiting neutrophil activation in retinal pigment epithelial cells comprising administering an inhibitor of IFNλ to a subject in need thereof.

The inhibitor can be any suitable inhibitor, such as a neutralizing anti-IFNλ antibody, an antisense oligonucleotide, a small interfering ribonucleic acid (siRNA), a small hairpin RNA (shRNA), a non-antibody binding polypeptide, or a small molecule chemical compound.

### Antibodies

The methods disclosed herein encompass inhibiting IFNλ by administering one or more neutralizing antibodies directed to IFNλ. The antibody includes at least one antibody selected from immunoglobulin, an antibody fragment, and an antibody analogue. The antibody may include any immunoglobulin that naturally occurs. The term "antibody fragment" includes an antibody fragment having a minimum unit of amino acid sequence that recognizes an antigen, and may include at least one antibody fragment selected from Fab, Fab', F(ab').sub.2, F(ab'), F(ab), Fv, and scFv. The term "antibody analogue" refers to an antibody that has a smaller protein molecular weight than an antibody fragment and that recognizes an antigen and binds thereto. In various embodiments, the antibody analogue may include at least one antibody analogue selected from affibody, repebody, affilin, DARPin, tetranectin, microbody, peptide aptamer, and avimer. When the antibody analogue is used, an antibody mimetic may be additionally included. In various embodiments, examples of the antibody mimetic are affilins, affimers, affitins, anticalins, avimers, and monobodies. The antibody mimetic may be manufactured in such a way that the antibody mimetic targets a particular receptor or antigen, and a manufacturing method thereof available herein may include a panning method. An antibody mimetic target factor has a smaller molecular mass than an antibody and a simple structure. The term "antibody" also refers to both monoclonal antibodies and polyclonal antibodies.

### RNA interference

The methods disclosed herein encompass inhibiting IFNλ by administering one or more nucleic acids selected from the group consisting of an antisense oligonucleotide, a small interfering ribonucleic acid (siRNA), and a small hairpin RNA (shRNA).

RNA interference (RNAi) is a process in which a dsRNA directs homologous sequence-specific degradation of messenger RNA. In mammalian cells, RNAi can be triggered by nucleotide duplexes of small interfering RNA (siRNA) without activating the host interferon response. The dsRNA can be a siRNA (containing two separate and complementary RNA chains) or a short hairpin RNA (i.e., a RNA chain forming a tight hairpin structure), both of which can be designed based on the sequence of the target gene. When the inhibitor is shRNA, the shRNA can target one or more of the nucleotide sequences consisting of the group consisting of SEQ ID NOs: 11-17.

Antisense oligonucleotide refers to a nucleic acid molecule complementary to a portion of a particular gene transcript that can hybridize to the transcript and block its translation. An antisense oligonucleotide can comprise RNA or DNA.

### Non-Antibody Binding Polypeptides

The methods disclosed herein encompass inhibiting IFNλ by administering one or more non-antibody binding polypeptides directed to IFNλ. Binding polypeptides may be chemically synthesized using known polypeptide synthesis methodology or may be prepared and purified using recombinant technology. Binding polypeptides are usually at least about 5 amino acids in length, alternatively at least about 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 amino acids in length or more, wherein such binding polypeptides that are capable of binding, preferably specifically, to IFNλ. Binding polypeptides may be identified without undue experimentation using well known techniques. In this regard, it is noted that techniques for screening polypeptide libraries for binding polypeptides that are capable of binding to a polypeptide target are well known in the art.

### Small Molecule Chemical Compounds

The methods disclosed herein encompass inhibiting IFNλ by administering one or more small molecule chemical compounds directed to directed to IFNλ. The small molecule chemical compound may be a component of a combinatorial chemical library. Combinatorial chemical libraries are a collection of multiple species of chemical compounds comprised of smaller subunits or monomers. Combinatorial libraries come in a variety of sizes, ranging from a few hundred to many hundreds of thousand different species of chemical compounds. There are also a variety of library types, including oligomeric and polymeric libraries comprised of compounds such as carbohydrates, oligonucleotides, and small organic molecules, etc. Such libraries have a variety of uses, such as immobilization and chromatographic separation of chemical compounds, as well as uses for identifying and characterizing ligands capable of binding an acceptor molecule (such as IFNλ) or mediating a biological activity of interest (such as, but not limited to, inhibition of neutrophil activation).

Various techniques for synthesizing libraries of compounds on solid-phase supports are known in the art. Solid-phase supports are typically polymeric objects with surfaces that are functionalized to bind with subunits or monomers to form the compounds of the library. Synthesis of one library typically involves a large number of solid-phase supports. To make a combinatorial library, solid-phase supports are reacted with one or more subunits of the compounds and with one or more numbers of reagents in a carefully controlled, predetermined sequence of chemical reactions.

Small molecules may be identified and chemically synthesized using known methodology. Small molecules are usually less than about 2000 Daltons in size or alternatively less than about 1500, 750, 500, 250 or 200 Daltons in size, wherein such small molecules that are capable of binding, preferably specifically, to IFNλ may be identified without undue experimentation using well known techniques. In this regard, it is noted that techniques for screening small molecule libraries for molecules that are capable of binding to a polypeptide target are well known in the art. Small molecules may be, for example, aldehydes, ketones, oximes, hydrazones, semicarbazones, carbazides, primary amines, secondary amines, tertiary amines, N-substituted hydrazines, hydrazides, alcohols, ethers, thiols, thioethers, disulfides, carboxylic acids, esters, amides, ureas, carbamates, carbonates, ketals, thioketals, acetals, thioacetals, aryl halides, aryl sulfonates, alkyl halides, alkyl sulfonates, aromatic compounds, heterocyclic compounds, anilines, alkenes, alkynes, diols, amino alcohols, oxazolidines, oxazolines, thiazolidines, thiazolines, enamines, sulfonamides, epoxides, aziridines, isocyanates, sulfonyl chlorides, diazo compounds, acid chlorides, or the like.

Polypeptide-containing IFNλ inhibitors (e.g., neutralizing antibody or non-antibody binding polypeptide) and the polypeptide comprising, consisting essentially of, or consisting of βA1-crystallin can be prepared by any of a number of conventional techniques. In this respect, the polypeptide sequence can be synthetic, recombinant, isolated, and/or purified.

The polypeptide can be isolated or purified from a recombinant source. For instance, a DNA fragment encoding a desired polypeptide can be subcloned into an appropriate vector using well-known molecular genetic techniques. The fragment can be transcribed and the polypeptide subsequently translated *in vitro.* Commercially available kits also can be employed. The polymerase chain reaction optionally can be employed in the manipulation of nucleic acids.

The polypeptide also can be synthesized using an automated peptide synthesizer in accordance with methods known in the art. Alternately, the polypeptide can be synthesized using standard peptide synthesizing techniques well-known to those of skill in the art. In particular, the polypeptide can be synthesized using the procedure of solid-phase synthesis. If desired, this can be done using an automated peptide synthesizer. Removal of the t-butyloxycarbonyl (t-BOC) or 9-fluorenylmethyloxycarbonyl (Fmoc) amino acid blocking groups and separation of the polypeptide from the resin can be accomplished by, for example, acid treatment at reduced temperature. The protein-containing mixture then can be extracted, for instance, with diethyl ether, to remove non-peptidic organic compounds, and the synthesized polypeptide can be extracted from the resin powder (e.g., with about 25% w/v acetic acid). Following the synthesis of the polypeptide, further purification (e.g., using HPLC) optionally can be performed in order to eliminate any incomplete proteins, polypeptides, peptides or free amino acids. Amino acid and/or HPLC analysis can be performed on the synthesized polypeptide to validate its identity. For other applications according to the invention, it may be preferable to produce the polypeptide as part of a larger fusion protein, either by chemical conjugation or through genetic means, such as are known to those skilled in the art. In this regard, the invention also provides a fusion protein comprising the polypeptide and one or more other protein(s) having any desired properties or functions, such as to facilitate isolation, purification, analysis, or stability of the fusion protein.

In addition to the IFNλ inhibitors, shRNA, siRNA, and antisense oligonucleotides, the invention provides a nucleic acid encoding the polypeptide comprising βA1-crystallin or the polypeptide-containing inhibitor (e.g., neutralizing antibody or non-antibody binding polypeptide). "Nucleic acid" as used herein includes "polynucleotide," "oligonucleotide," and "nucleic acid molecule," and generally means a polymer of DNA or RNA, which can be single-stranded or double-stranded, synthesized or obtained (e.g., isolated and/or purified) from natural sources, which can contain natural, non-natural or altered nucleotides, and which can contain a natural, non-natural or altered intemucleotide linkage, such as a phosphoroamidate linkage or a phosphorothioate linkage, instead of the phosphodiester found between the nucleotides of an unmodified oligonucleotide. It is generally preferred that the nucleic acid does not comprise any insertions, deletions, inversions, and/or substitutions. However, it may be suitable in some instances, as discussed herein, for the nucleic acid to comprise one or more insertions, deletions, inversions, and/or substitutions.

In an embodiment, the nucleic acid is recombinant. As used herein, the term "recombinant" refers to (i) molecules that are constructed outside living cells by joining natural or synthetic nucleic acid segments to nucleic acid molecules that can replicate in a living cell, or (ii) molecules that result from the replication of those described in (i) above. For purposes herein, the replication can be *in vitro* replication or *in vivo* replication.

The nucleic acid (e.g., DNA, RNA, cDNA, and the like) can be produced in any suitable matter including, but not limited to recombinant production and commercial synthesis. In this respect, the nucleic acid sequence can be synthetic, recombinant, isolated, and/or purified.

The nucleic acid can be constructed based on chemical synthesis and/or enzymatic ligation reactions using procedures known in the art. See, for example, Green et al. (eds.), Molecular Cloning, A Laboratory Manual, 4th Edition, Cold Spring Harbor Laboratory Press, New York (2012). For example, a nucleic acid can be chemically synthesized using naturally occurring nucleotides or variously modified nucleotides designed to increase the biological stability of the molecules or to increase the physical stability of the duplex formed upon hybridization (e.g., phosphorothioate derivatives and acridine substituted nucleotides). Examples of modified nucleotides that can be used to generate the nucleic acids include, but are not limited to, 5-fluorouracil, 5-bromouracil, 5-chlorouracil, 5-iodouracil, hypoxanthine, xanthine, 4-acetylcytosine, 5-(carboxyhydroxymethyl) uracil, 5-carboxymethylaminomethyl-2-thiouridine, 5-carboxymethylaminomethyluracil, dihydrouracil, beta-D-galactosylqueosine, inosine, N⁶-isopentenyladenine, 1-methylguanine, 1-methylinosine, 2,2-dimethylguanine, 2-methyladenine, 2-methylguanine, 3-methylcytosine, 5-methylcytosine, N⁶-substituted adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N⁶-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, 3-(3-amino-3-N-2-carboxypropyl) uracil, and 2,6-diaminopurine. Alternatively, one or more of the nucleic acids of the invention can be purchased from companies, such as Macromolecular Resources (Fort Collins, CO) and Synthegen (Houston, TX).

The nucleic acid can be provided as part of a construct comprising the nucleic acid and elements that enable delivery of the nucleic acid to a cell, and/or expression of the nucleic acid in a cell. For example, the polynucleotide sequence corresponding to the IFNλ inhibitors shRNA, siRNA, and antisense oligonucleotides or the polynucleotide sequence encoding the polypeptide comprising βA1-crystallin or polypeptide-containing inhibitor can be operatively linked to expression control sequences. An expression control sequence operatively linked to a coding sequence is ligated such that expression of the coding sequence is achieved under conditions compatible with the expression control sequences. The expression control sequences include, but are not limited to, appropriate promoters, enhancers, transcription terminators, a start codon (i.e., ATG) in front of a protein-encoding gene, splicing signal for introns, maintenance of the correct reading frame of that gene to permit proper translation of mRNA, and stop codons. Suitable promoters include, but are not limited to, a hVMD2 promoter, an Aldh1L1 promoter, an SV40 early promoter, RSV promoter, adenovirus major late promoter, human CMV immediate early I promoter, poxvirus promoter, 30K promoter, I3 promoter, sE/L promoter, 7.5K promoter, 40K promoter, and C1 promoter.

The nucleic acid can be cloned or amplified by *in vitro* methods, such as the polymerase chain reaction (PCR), the ligase chain reaction (LCR), the transcription-based amplification system (TAS), the self-sustained sequence replication system (3SR) and the Qβ replicase amplification system (QB). For example, a polynucleotide encoding the polypeptide can be isolated by polymerase chain reaction of cDNA using primers based on the DNA sequence of the molecule. A wide variety of cloning and in vitro amplification methodologies are well known to persons skilled in the art.

The invention provides a vector comprising the nucleic acid. The nucleic acid can be inserted into any suitable vector. The selection of vectors and methods to construct them are commonly known in the art and are described in general technical references.

Suitable vectors include those designed for propagation and expansion or for expression or both. Examples of suitable vectors include, for instance, plasmids, plasmid-liposome complexes, CELid vectors (see, e.g., Li et al., PLoS One., 8(8): e69879. doi: 10.1371/journal.pone.0069879 (2013)) and viral vectors, e.g., parvoviral-based vectors (i.e., AAV vectors), retroviral vectors, herpes simplex virus (HSV)-based vectors, adenovirus-based vectors, and poxvirus vectors. Any of these expression constructs can be prepared using standard recombinant DNA techniques.

In one aspect of the invention, the vector is a viral vector, such as an AAV vector. The AAV vector may be suitable for packaging into any AAV serotype or variant thereof that is suitable for administration to ocular cells (e.g., RPE cells). Examples of suitable AAV serotypes may include, but are not limited to, AAV1, AAV2, AAV3, AAV4, AAV5, AAV6, AAV7, AAV8, AAV9, variants thereof, and engineered or newly synthesized AAV viruses such as AAV.7m8.

The AAV vector may be packaged in a capsid protein, or fragment thereof, of any of the AAV serotypes described herein. Preferably, the vector is packaged in an AAV2 or AAV8 capsid.

A suitable recombinant AAV may be generated by culturing a packaging cell which contains a nucleic acid sequence encoding an AAV serotype capsid protein, or fragment thereof, as defined herein; a functional *rep* gene; any of the inventive vectors described herein; and sufficient helper functions to permit packaging of the inventive vector into the AAV capsid protein. The components required by the packaging cell to package the inventive AAV vector in an AAV capsid may be provided to the host cell in *trans.* Alternatively, any one or more of the required components (e.g., inventive vector, *rep* sequences, capsid sequences, and/or helper functions) may be provided by a stable packaging cell which has been engineered to contain one or more of the required components using methods known to those of skill in the art.

In an embodiment of the invention, the AAV vector is self-complementary. Self-complementary vectors may, advantageously, overcome the rate-limiting step of second-strand DNA synthesis and confer earlier onset and stronger gene expression.

In an embodiment of the invention, the vector is a recombinant expression vector. For purposes herein, the term "recombinant expression vector" means a genetically-modified oligonucleotide or polynucleotide construct that permits the expression of an mRNA, protein, polypeptide, or peptide by a host cell, when the construct comprises a nucleotide sequence encoding the mRNA, protein, polypeptide, or peptide, and the vector is contacted with the cell under conditions sufficient to have the mRNA, protein, polypeptide, or peptide expressed within the cell. The vectors of the invention are not naturally-occurring as a whole. However, parts of the vectors can be naturally-occurring. The inventive recombinant expression vectors can comprise any type of nucleotides, including, but not limited to DNA and RNA, which can be single-stranded or double-stranded, synthesized or obtained in part from natural sources, and which can contain natural, non-natural or altered nucleotides. The recombinant expression vectors can comprise naturally-occurring, non-naturally-occurring intemucleotide linkages, or both types of linkages. Preferably, the non-naturally occurring or altered nucleotides or internucleotide linkages do not hinder the transcription or replication of the vector.

The vector can be prepared using standard recombinant DNA techniques described in, for example, Green et al., *supra.* Constructs of expression vectors, which are circular or linear, can be prepared to contain a replication system functional in a prokaryotic or eukaryotic host cell. Replication systems can be derived, e.g., from ColEl, 2 µ plasmid, λ, SV40, bovine papilloma virus, and the like
In addition to the nucleic acid described above, the vector can comprise one or more nucleic acid sequences encoding one or more polypeptides for delivery and expression in a subject/host (e.g., a mammal, such as a mouse, rat, guinea pig, hamster, cat, dog, rabbit, pig, cow, horse, or primate (e.g., human)).

The vector can include one or more marker genes, which allow for selection of transformed or transfected hosts. Marker genes include biocide resistance, e.g., resistance to antibiotics, heavy metals, etc., complementation in an auxotrophic host to provide prototrophy, and the like. Suitable marker genes for the inventive expression vectors include, for instance, neomycin/G418 resistance genes, hygromycin resistance genes, histidinol resistance genes, tetracycline resistance genes, and ampicillin resistance genes.

The vector may further comprise regulatory sequences that permit one or more of the transcription, translation, and expression of nucleic acid comprised in the vector in a cell transfected with the vector or infected with a virus that comprises the vector. For example, "operably linked" sequences include both regulatory sequences that are contiguous with the nucleic acid of interest (e.g., the nucleotide sequence encoding βA1-crystallin) and regulatory sequences that act in *trans* or at a distance to control the nucleotide sequence (e.g., the nucleotide sequence encoding βA1-crystallin polypeptide).

The regulatory sequences may include appropriate transcription initiation, termination, promoter and enhancer sequences; RNA processing signals such as splicing and polyadenylation (polyA) signal sequences; sequences that stabilize cytoplasmic mRNA; sequences that enhance translation efficiency (i.e., Kozak consensus sequence); sequences that enhance protein stability; and when desired, sequences that enhance secretion of the encoded product. PolyA signal sequences may be synthetic or may be derived from many suitable species, including, for example, SV-40, human and bovine. Figs. 8 and 9 provide an exemplary vector map of a vector comprising the nucleotide sequence of βA1-crystallin (scCAG_humCryba1).

When the vector is for administration to a host (e.g., human), the vector (e.g., AAV) preferably has a low replicative efficiency in a target cell (e.g., no more than about 1 progeny per cell or, more preferably, no more than 0.1 progeny per cell are produced). Replication efficiency can readily be determined empirically by determining the virus titer after infection of the target cell.

The polypeptide, nucleic acid, vector, or inhibitor (e.g., small molecule) can be formulated as a composition (e.g., pharmaceutical composition) comprising the polypeptide, nucleic acid, vector, or inhibitor and a carrier (e.g., a pharmaceutically or physiologically acceptable carrier). Furthermore, the polypeptide, nucleic acid, vector, inhibitor or composition of the invention can be used in the methods described herein alone or as part of a pharmaceutical formulation.

The composition (e.g., pharmaceutical composition) can comprise more than one polypeptide, nucleic acid, vector, inhibitor (e.g., small molecule), or composition of the invention. Alternatively, or in addition, the composition can comprise one or more other pharmaceutically active agents or drugs. Examples of such other pharmaceutically active agents or drugs that may be suitable for use in the pharmaceutical composition include lampalizumab (anti-complement factor D; Genentech) for patients with geographic atrophy secondary to AMD); brolicizumab (pan-isoform ant-VEGF-A; Novartis) for wet AMD; OPT-302 (soluble VEGF-C/D receptor; Ophthea) for wet AMD and diabetic retinal edema (DME); PanOptica's topical VEGF inhibitor for wet AMD; pegpleranib (DNA aptamer binding to PDGF isoforms; Ophtotech/Novartis) optionally combined with LUCENTIS^{™} (ranibizumab injection); rinucumab (anti-PDGF receptor; Regeneron) optionally co-formulated with EYLEA^{™} (aflibercept); DE-120 (anti-PDGF/VEGF bispecific; Santen); vorolanib (oral RTK inhibitor that inhibits kinase activity for pDGF and VEGF; Tyrogenex); nevacumab (anti-angiopoeitin 2; Regeneron) optionally combined with EYLEA^{™} (aflibercept); RG-7716 (anti-angiopoeitin 2/VEGF bispecific; Chugai); ARP-1536 (anti VE PTP; AkebialAeripo); ICON-1 (chimeric protein binding to Tissue Factor; Iconic Therapeutic); and carotuximab (anti-endogin; Tracon/Santen). Additionally or alternatively, the composition can comprise inhibitors of PTP1B.

The carrier can be any of those conventionally used and is limited only by physio-chemical considerations, such as solubility and lack of reactivity with the active compound(s) and by the route of administration. The pharmaceutically acceptable carriers described herein, for example, vehicles, adjuvants, excipients, and diluents, are well-known to those skilled in the art and are readily available to the public. It is preferred that the pharmaceutically acceptable carrier be one which is chemically inert to the active agent(s) and one which has no detrimental side effects or toxicity under the conditions of use.

The choice of carrier will be determined in part by the particular polypeptide, nucleic acid, vector, inhibitor (e.g., small molecule), or composition thereof of the invention and other active agents or drugs used, as well as by the particular method used to administer the polypeptide, nucleic acid, vector, inhibitor, or composition thereof.

The polypeptide, nucleic acid, vector, inhibitor (e.g., small molecule), or composition thereof can be administered to the subject by any method. For example, the polypeptide, nucleic acid, or vector can be introduced into a cell (e.g., in a subject) by any of various techniques, such as by contacting the cell with the nucleic acid or the vector as part of a construct, as described herein, that enables the delivery and expression of the nucleic acid. Specific protocols for introducing and expressing nucleic acids in cells are known in the art.

Any suitable dose of the polypeptide, nucleic acid, vector, inhibitor (e.g., small molecule), or composition thereof can be administered to a subject. The appropriate dose will vary depending upon such factors as the subject's age, weight, height, sex, general medical condition, previous medical history, and disease progression, and can be determined by a clinician. The amount or dose should be sufficient to effect the desired biological response, e.g., a therapeutic or prophylactic response, in the subject over a clinically reasonable time frame.

For example, the polypeptide can be administered in a dose of about 0.05 mg to about 10 mg (e.g., 0.1 mg, 0.5 mg, 1 mg, 2 mg, 3 mg, 4 mg, 5 mg, 6 mg, 7 mg, 8 mg, 9 mg, and ranges thereof) per vaccination of the host (e.g., mammal, such as a human), and preferably about 0.1 mg to about 5 mg per vaccination. Several doses (e.g., 1, 2, 3, 4, 5, 6, or more) can be provided (e.g., over a period of weeks or months).

The dosing period may be appropriately determined depending on the therapeutic progress. In embodiments, the dosing period may comprise less than one year, less than 9 months, less than 8 months, less than 7 months, less than 6 months, less than 5 months, less than 4 months, less than 3 months, less than 2 months, or one month. In other embodiments, the dosing period may comprise three doses per day, two doses per day, or one dose per day for the length of the dosing period.

When the vector is a viral vector, a suitable dose can include about 1 × 10⁵ to about 1 × 10¹² (e.g., 1 × 10⁶, 1 × 10⁷, 1 × 10⁸, 1 × 10⁹, 1 × 10¹⁰, 1 × 10¹¹, and ranges thereof) plaque forming units (pfus), although a lower or higher dose can be administered to a host.

The polypeptide, nucleic acid, vector, inhibitor (e.g., small molecule), or composition thereof can be administered to the subject by various routes including, but not limited to, topical, subcutaneous, intramuscular, intradermal, intraperitoneal, intrathecal, intravenous, subretinal injection, and intravitreal injection. In one embodiment, the polypeptide, nucleic acid, vector, or composition can be directly administered (e.g., locally administered) by direct injection into the eye by subretinal or inravitreal injection or by topical application (e.g., as eye drops). When multiple administrations are given, the administrations can be at one or more sites in a subject and a single dose can be administered by dividing the single dose into equal portions for administration at one, two, three, four or more sites on the individual.

The invention further provides a method of rejuvenating Vacuolar-type H⁺-ATPase (V-ATPase) activity in RPE cells by modulating assembly and disassembly of the V-ATPase in a subject in need thereof. The assembly and disassembly of the V-ATPase holoenzyme can be modulated by any suitable means including by targeting specific residues or peptides of V-ATPase (e.g., residues that interact with βA3/A1-crystallin). Fig. 10 provides a schematic showing that βA3/A1-crystallin binds to V-ATPase in the lysosomal lumen, as well as describing residue boundaries for portions of the rat V-ATPase holoenzyme. The rat and human V-ATPase sequences are highly conserved. Modulating assembly and disassembly of the V-ATPase holoenzyme (e.g., by targeting specific residues of V-ATPase for mutation) can rescue the proper functioning of RPE in disorders such as dry AMD.

In one embodiment, the subject has age-related macular degeneration (AMD) or is at risk for developing AMD. The AMD can be wet AMD or atrophic (dry) AMD. The subject may have geographic atrophy secondary to AMD. As such, the invention also provides a method of treating or preventing AMD in the subject.

In one embodiment, the subject has Stargardt's macular retinal degeneration or is at risk for developing Stargardt's macular retinal degeneration. As such, the invention also provides a method of treating or preventing Stargardt's macular retinal degeneration.

In another embodiment, the subject has a neurodegenerative disease or is at risk for developing a neurodegenerative disease, such as Alzheimer's disease or Parkinson's disease. As such, the invention also provides a method of treating or preventing a neurodegenerative disease, such as Alzheimer's disease or Parkinson's disease, in the subject.

In a further embodiment, the subject has diabetic retinopathy (DR) or is at risk for developing DR. The subject also may have diabetic macular edema (DME) or be at risk for developing DME. As such, the invention also provides a method of treating or preventing DR or DME in the subject.

Administration of the polypeptide, nucleic acid, vector, inhibitor (e.g., small molecule), or composition thereof can be "prophylactic" or "therapeutic." When provided prophylactically, the polypeptide, nucleic acid, vector, inhibitor (e.g., small molecule), or composition thereof is provided in advance of a subject's diagnosis with AMD, neurodegenerative disease, DR, and/or DME. For example, subjects at risk for developing AMD, Stargardt's macular retinal degeneration, neurodegenerative disease, DR, and/or DME are a preferred group of patients treated prophylactically. The prophylactic administration of the polypeptide, nucleic acid, vector, inhibitor (e.g., small molecule), or composition thereof prevents, ameliorates, or delays AMD, Stargardt's macular retinal degeneration, neurodegenerative disease, DR, and/or DME. When provided therapeutically, the polypeptide, nucleic acid, vector, or composition thereof is provided at or after the diagnosis of AMD, Stargardt's macular retinal degeneration, neurodegenerative disease, DR, and/or DME.

When the subject has already been diagnosed with AMD, Stargardt's macular retinal degeneration, neurodegenerative disease, DR, or DME, the polypeptide, nucleic acid, vector, or composition thereof can be administered in conjunction with other therapeutic treatments such as lampalizumab (anti-complement factor D; Genentech); brolicizumab (pan-isoform ant-VEGF-A; Novartis); OPT-302 (soluble VEGF-C/D receptor; Ophthea); PanOptica's topical VEGF inhibitor; pegpleranib (DNA aptamer binding to PDGF isoforms; Ophtotech/Novartis); LUCENTIS^{™} (ranibizumab injection); rinucumab (anti-PDGF receptor; Regeneron); EYLEA^{™} (aflibercept); DE-120 (anti-PDGF/VEGF bispecific; Santen); vorolanib (oral RTK inhibitor that inhibits kinase activity for pDGF and VEGF; Tyrogenex); nevacumab (anti-angiopoeitin 2; Regeneron); RG-7716 (anti-angiopoeitin 2/VEGF bispecific; Chugai); ARP-1536 (anti VE PTP; AkebialAeripo); ICON-1 (chimeric protein binding to Tissue Factor; Iconic Therapeutic); and/or carotuximab (anti-endogin; Tracon/Santen).

The terms "treat," and "prevent," as used herein, do not necessarily imply 100% or complete treatment or prevention. Rather, there are varying degrees of treatment or prevention of which a person of ordinary skill in the art recognizes as having a potential benefit or therapeutic effect.

There are a variety of suitable formulations of the pharmaceutical composition for the inventive methods. Any suitable carrier can be used within the context of the invention, and such carriers are well known in the art. The choice of carrier will be determined, in part, by the particular site to which the composition is to be administered (e.g., ocular cells, RPE cells, photoreceptor cells, rods, and cones) and the particular method used to administer the composition. The pharmaceutical composition can optionally be sterile or sterile with the exception of the one or more adeno-associated viral vectors.

Suitable formulations for the pharmaceutical composition include aqueous and non-aqueous solutions, isotonic sterile solutions, which can contain anti-oxidants, buffers, and bacteriostats, and aqueous and non-aqueous sterile suspensions that can include suspending agents, solubilizers, thickening agents, stabilizers, and preservatives. The formulations can be presented in unit-dose or multi-dose sealed containers, such as ampules and vials, and can be stored in a freeze-dried (lyophilized) condition requiring only the addition of the sterile liquid carrier, for example, water, immediately prior to use. Extemporaneous solutions and suspensions can be prepared from sterile powders, granules, and tablets. Preferably, the carrier is a buffered saline solution. More preferably, the pharmaceutical composition for use in the inventive method is formulated to protect the polypeptide, nucleic acid, or vector from damage prior to administration. For example, the pharmaceutical composition can be formulated to reduce loss of the polypeptide, nucleic acid, or vector on devices used to prepare, store, or administer the polypeptide, nucleic acid, or vector, such as glassware, syringes, or needles. The pharmaceutical composition can be formulated to decrease the light sensitivity and/or temperature sensitivity of the polypeptide, nucleic acid, or vector. To this end, the pharmaceutical composition preferably comprises a pharmaceutically acceptable liquid carrier, such as, for example, those described above, and a stabilizing agent selected from the group consisting of polysorbate 80, L-arginine, polyvinylpyrrolidone, trehalose, and combinations thereof. Use of such a composition may extend the shelf life of the polypeptide, nucleic acid, vector, or inhibitor (e.g., small molecule), facilitate administration, and increase the efficiency of the inventive method.

A pharmaceutical composition also can be formulated to enhance transduction/transfection efficiency of the polypeptide, nucleic acid, vector, or inhibitor (e.g., small molecule). In addition, a person of ordinary skill in the art will appreciate that the pharmaceutical composition can comprise other therapeutic or biologically-active agents.

The following examples further illustrate the invention but, of course, should not be construed as in any way limiting its scope.

### EXAMPLE 1

This example provides materials and methods for the following Examples.

### Antibodies

PE/Cy7-tagged CD45 (Cat# 103114), APC-tagged Ly6C (Cat# 128016), FITC-tagged CD66b (Cat# 555724), V450-tagged Ly6G (Cat# 560603), Alexa fluor 700-tagged CD11b (Cat# 557960), anti-human PE/Cy7-tagged CD45 (Cat# 560178), and Anti human CD34 antibody (Cat# 343602) were purchased from BD Biosciences, USA and anti-human PE-tagged IL-28AR antibody (Cat# 337804) was purchased from Biolegend, USA. Anti-Neutrophil Elastase (Cat# ab68672), anti-GRO alpha (CXCL1) (Cat# ab86436), anti-STAT1 (phosphor S727) (Cat# ab109461), anti-IL28 receptor alpha or IL28R1 (Cat # ab224395), anti-Histone H3 citrunillated (Cat# ab219407), VCAM1 (Cat# ab134047), CD34 (Cat# 8158) and IL28 + IL29 (Cat# ab191426) antibodies were purchased from Abcam, USA. Anti-ICAM-1 (Cat# SC-107), Anti-STAT1 (Cat# 9172T), anti-AKT (Cat# 4685S), anti-AKT2 (Cat# 2964S) and anti-DAB2 (Cat# 12906S) were purchased from Cell Signaling Technologies, USA. Other antibodies used include: Alexa fluor 488-tagged β1 Integrin (Santa Cruz Biotechnology, USA; Cat# sc-374429 AF488), Anti-IL-28A/IFNλ2 (Antibodies online, USA; Cat# ABIN357173), Anti-Ly6G (Antibodies online, USA; Cat# ABIN1854937), IL-29 antibody (Biorbyt, USA; Cat# orb6201), anti-IFNα (Thermo Fisher, USA; Cat# 221001), anti-Myeloperoxidase/MPO (R&D Systems, USA; Cat# AF3667-SP), anti-LCN-2 (EMD Milipore; Cat# AB2267) and anti-Actin (Sigma Aldrich, USA; Cat# A2066).

### Animals

Both male and female βA3/A1-crystallin conditional knockout mice (*Cryba1* cKO C57B1/6J mice) and LCN-2 KO C57B1/6J mice were generated as described previously explained (Valapala et al., Aging Cell, 13: 1091-1094 (2014); and Berger et al., Proc. Natl. Acad. Sci. U.S.A., 103: 1834-1839 (2006)). NOD-SCID mice (NOD.CB17-Prkdescid/J; 4-5 weeks old) were purchased from The Jackson Laboratory, USA. All animal studies were conducted in accordance with the Guide for the Care and Use of Animals (National Academy Press) and were approved by the University of Pittsburgh Animal Care and Use Committee.

### Human Eyes

The diagnosis and classification of AMD in human donor eyes was done as previously described (Ghosh et al., J. Pathol., 241: 583-588 (2017)). For immunostaining, human donor eyes were obtained from the National Disease Research Interchange (NDRI; Philadelphia, Pennsylvania, USA) within 12-35 h of death. Donor eyes from 5 subjects with early, dry AMD (age range 79-95 years; mean age 85.8 years) and three aged controls (age range 77-89 years; mean age 82.5 years), with no evidence of macular disease were studied (Ghosh et al., *supra*)*.* The study adhered to the norms of the Declaration for Helsinki regarding research involving human tissue. For immunophenotyping and soluble factors quantification experiments in human peripheral blood and aqueous humor, samples were collected from human donors, reporting to Narayana Nethralaya, Bangalore, India. All subjects underwent an ophthalmic exam, including visual acuity testing and retinal examination. Early AMD patients were diagnosed by fundus imaging, Amsler grid test and OCT imaging when deemed necessary and classified as per the (Age-Related Eye Disese Study Research Group (AREDS). Subjects with co-existing glaucoma or any other degenerative retinal disorders were excluded. The control group consisted of individuals without any history of AMD, diabetes, cardiovascular disorders, or retinal diseases. 4-6 mL blood samples were collected in EDTA tubes from 18 controls and 43 AMD subjects by venipuncture. Aqueous humor samples (~50 µL) were collected from subjects undergoing cataract surgery (n=7 control, n=6 AMD) by anterior chamber paracentesis under sterile conditions. Within this group, early AMD subjects, where surgery is not contra-indicated, were identified by the presence of drusen and RPE abnormalities characterized by pigmentary changes in the retina in accordance with AREDS classification. All collected samples were immediately stored in a biorepository until further processing. All patient samples and related clinical information were collected after obtaining approval by the Narayana Nethralaya Institutional Review Board (IRB) and with written, informed consent from patients.

### Immunostaining

Freshly enucleated eyes were fixed in 2% paraformaldehyde (PFA) for 10 min and then the anterior parts (cornea, lens, and attached iris pigmented epithelium) were removed. The resulting posterior eyecups were fixed in 2% PFA for 1 h at room temperature either for cryosections or RPE/retina flat mount. For cryosections, the eyecups were dehydrated through gradient sucrose solutions and embedded in OCT and for RPE/retina flatmounts, tissues were removed after the eyecup was quartered like a petaloid structure. The resulting eyecup was further cut radially into eight pieces from the optic nerve head to the periphery. Immunostaining on human/mouse retina sections or on retinalRPE flatmounts were performed by using appropriate primary antibody (1:100) and incubated at 4 °C overnight. The RPE/retina flatmounts or human or mouse retina sections were washed with 1X TBS thrice and then stained with appropriate secondary antibodies (1:300) with 1 µg/mL DAPI (Sigma Aldrich, USA) in the dark at room temperature for 2 h. The tissue sections or flatmounts were washed 6 times with 1X TBS. The tissues were mounted on a cover slip with DAKO mounting agent and then visualized under a confocal microscope (Zeiss LSM710, Switzerland).

### Soluble factors quantification

Peripheral venous blood was obtained by venipuncture (n=43 AMD patients and n=18 control subjects) and aqueous humor (AH) was collected by anterior chamber paracentesis in AMD patients (n=6) and control subjects (n=7) from subjects undergoing cataract surgery. The levels of IFNα, IFNβ, IFNγ, IFNλ1-3, VEGF, and CXCL1 were measured in plasma and AH by bead-based multiplex ELISA (BioLegend, Inc, USA) using a flow cytometer (BD FACS Canto II, FACS DIVA software, BD Biosciences, USA). The absolute concentration for each analyte was calculated based on the standard curve using LEGENDplex^{™} software (Biolegend, Inc, USA).

### Immunophenotyping

Cells from peripheral blood (n=43 AMD patients and n=18 control subjects) were labeled using fluorochrome conjugated anti-human antibodies specific for leukocytes (APC-Cy7-tagged CD45), neutrophils (FITC-tagged CD66b) and IFNλ receptor (PE-tagged IL-28R1) at room temperature for 45 minutes. Red blood cells from peripheral blood samples were lysed in 1X BD lysis buffer for 10 minutes, washed, and resuspended in 1X phosphate buffered saline prior to flow cytometry (BD FACS Canto II, FACS DIVA software, BD Biosciences, USA) based acquisition and analysis. Data were analyzed using FCS Express 6 Flow Research Edition software. The leukocyte populations were identified by manual gating using SSC/CD45⁺ profile. Subsequent gating was done on SSC/CD66b FITC to identify neutrophils. The neutrophil activation status was determined based on CD66b cell surface expression. CD45⁺CD66b^{high} cells were considered as activated neutrophils and CD45⁺CD66b^{low} as inactive neutrophils. CD45⁺CD66b^{high/low} IL-28R1⁺ indicated IFNλ receptor positive neutrophils. The percentage of positive cell events for each staining panel was calculated.

### RPE isolation and culture

Mouse RPE was isolated from control C57BL/6J mice (3 weeks old, n=9; Jackson Laboratories, USA) and cultured by enucleating the eyes and then washed twice in DMEM containing high glucose and incubated in 2% (weight/volume) Dispase (Roche, 10269638001) in DMEM for 45 min at 37°C. The eyes were then washed twice in growth medium made of DMEM (high glucose) containing 10% FCS, 1% penicillin/streptomycin, 2.5 mM L-glutamine, and 1X MEM nonessential amino acids (Gibco, Invitrogen, 11095). An incision was made around the ora serrata of each eye and the anterior segment was removed. The resulting posterior eyecups were placed in growth medium for 20 min at 37 °C to initiate separation of the neural retina from the RPE. The neural retina was removed and intact sheets of RPE cells were peeled off the underlying Bruch's membrane and transferred in a sterile 60-mm culture dish, containing fresh growth medium. The RPE sheets were washed thrice with growth medium and then twice with calcium and magnesium free HBSS and then briefly triturated, using a fine point Pasteur pipette. RPE cells were centrifuged at 200 g for 5 min and cultured in transwell plates in growth medium.

### IFNλ overexpression in cultured RPE cells

pLV-C-IL28A-GFPSpark and control vector were purchased from Sino Biological Inc. (Beijing, China, Cat# MG51305-ACGLN). Primary mouse RPE cells (in a monolayer; 90% confluent) were transfected with the respective vectors using X-tremeGENE transfection reagent (Roche, Switzerland) following the manufacturer's instructions. The transfection efficiency was estimated by evaluating the level of IL-28A/IFNλ released (into the cell-free supernatant) from overexpression transfected RPE cells by ELISA, with respect to the control vector transfected cells; a minimum of a three-fold increase in IL-28A/IFNλ level was considered appropriate for performing further experiments with the conditioned media.

### Isolation and culture of neutrophils

Neutrophils from WT and LCN-2^{-/-} mice were isolated by centrifugation of bone marrow cells, flushed from femurs and tibias, and purified over a Percoll discontinuous density gradient following isolation, neutrophils were resuspended at a density of 10 X 10⁶ per ml in Ca²⁺ and Mg²⁺ free HBSS, supplemented with 20 mM HEPES and then cultured in 37°C at a density of 3 × 10⁶ cells per ml before stimulation with either recombinant IFNλ or conditioned media from RPE cells overexpressing IFNλ.

### pHrodo phagocytic assay

Neutrophils in culture were incubated with fluorescent-tagged particles (pHrodo^{™} Red E. coli BioParticles^{™} Conjugate for Phagocytosis assay kit, Thermo Fisher, USA, Cat# P35361) and flow cytometric evaluation of percentage cells which has engulfed the pHrodo particles (phagocytic cells) was performed by following the manufacturer's protocol.

### Integrin β1 shRNA transfection

Integrin β1 shRNA lentiviral (Cat# sc-60044-V) and control shRNA (Cat# sc-108080) particles were purchased from Santa Cruz Biotechnology, USA. Mouse bone marrow derived neutrophils (5 × 10⁶ cells/mL in HBSS containing 20 mM HEPES) were plated and then transfected with integrin β1 shRNA lentiviral or control shRNA particles for 8 h, according to the manufacturer's protocol.

### Rapid neutrophil adhesion assay

Mouse bone marrow derived neutrophils (5 × 10⁶ cells/mL in HBSS containing 20 mM HEPES) from LCN-2^{-/-} mice and WT mice respectively or neutrophils transfected with either control shRNA or integrin β1 shRNA were subjected to rapid adhesion assay. Glass bottom 35 mm plates were coated for 16 h at 4°C with human fibrinogen (20 µg/well in endotoxin-free PBS). Neutrophils from all experimental conditions (10⁵ per well; 5 × 10⁶ per mL in 10% FCS, 1 mM CaCl₂/MgCl₂ in PBS, pH 7.2) were added, incubated for 10 min at 37°C, and then fixed on ice in 1.5% glutaraldehyde for 60 min and then counted with computer assisted enumeration.

### Neutrophil transmigration assay

Neutrophils (5 × 10⁶ cells/mL in HBSS containing 20 mM HEPES medium) from LCN-2^{-/-} and WT mice respectively or neutrophils transfected with either control shRNA or integrin β1 shRNA were used to assess cell migration by using transwell plates. Neutrophils were plated on transwell inserts at 5 × 10⁶ cells per ml and then exposed to different experimental conditions and cultured at 37 °C. The cells at the bottom of the transwell were fixed with 1.5% glutaraldehyde for 60 minutes, stained with Giemsa and then counted with computer assisted enumeration.

### Estimation of percentage neutrophils in mouse retina

Mouse retinas were dissected from enucleated eyes and digested with 0.05% collagenase D (Roche, Switzerland, Cat# 11088858001) at 37 °C for 30 min, teased with blunt end forceps and pipetted to release cells, passed through a 70 µm cell strainer, and centrifuged at 1,300g, 4 °C for 20 minutes. The entire pellet was used for assessing the % neutrophils by flow cytometry, after staining with anti-Ly6G, Ly6C, CD11b and CD45 antibodies at a concentration of 1 µg/mL for 90 minutes at room temperature.

### Intracellular reactive oxygen species (ROS)

Flow cytometry was performed to evaluate the intracellular ROS in neutrophils by staining cells (1 × 10⁶ cells) from each experimental group with 2',7'-dichlorofluorescin diacetate (DCFDA, Sigma Aldrich, USA, Cat# D6883-50MG) (25 µg/ml) for 30 min at 37 °C. Excess DCFDA was washed and cells were resuspended in PBS. The ROS content of the cells was measured on a flow cytometer.

### Estimation of intracellular and extracellular expression of integrin β1

Freshly cultured bone marrow-derived neutrophils from WT and LCN-2^{-/-} mice were incubated with Alexa fluor 488-tagged β1-Integrin (Santa Cruz Biotechnology, USA) antibodies at a concentration of 1 µg/mL in PBS containing 1% BSA for 1 h and the cell surface expression of integrin β1 (FITC fluorescence) was evaluated among these cells as described previously (Teckchandani et al., J. Cell Biol., 186, 99-111 (2009)). For intracellular expression of integrin β1, cells were permeabilized with 0.1% Triton X-100 in PBS for 5 min at 25 °C before incubating with anti-integrin β1 antibody at a concentration of 1 µg/mL in PBS containing 1% BSA for 1 h. Cell were analyzed by flow cytometry.

### SDS-PAGE and western blot analysis

SDS-PAGE and western blot analyses were performed by suspending and sonicating cells or tissue samples in RIPA lysis buffer (Millipore, Billerica, MA, 20-188) plus 1% protease and phosphatase inhibitors (Sigma). Samples were placed on ice for 20 min and then centrifuged at 13,000 g for 20 min in 4 °C. The supernatants were subjected to protein estimation by BCA kit (Thermo Fisher, USA). 12 µg of protein was used per sample and mixed with 4X protein sample buffer (Invitrogen, Carlsbad, CA) with 5% 2-mercaptoethanol (Sigma Aldrich, USA) and heated at 100 °C for 10 min. Samples were loaded into a 4-12% Bis-Tris Nu-PAGE gel (Invitrogen), electrophoresis was performed in MES buffer (Novex, Waltham, MA, USA). Proteins were transferred to nitrocellulose membranes and blocked with 5% skim milk (Biorad, USA) or 5% BSA (Sigma, for phosphorylated proteins). The primary antibodies were used at a dilution of 1:1000 whereas, all secondary antibodies were used at a dilution of 1:3000.

### Preparation of recombinant lipocalin-2 (LCN-2) protein

Full length LCN-2 cDNA was synthesized by GeneScipt, USA. It was subcloned in pET28a vector at NdeI and XhoI restriction site. The construct was transformed into *E.coli* DH5-α cells for amplification and *E.coli* Rosetta for expression. A single colony was grown overnight as a mother culture. 10% of mother culture was inoculated and grown to 0.8-1.0 OD and induced with 0.5 mM IPTG for 2 h at 37° C. The cells were then pelleted by centrifugation at 6000 rpm for 10 minutes at 4 °C in a microfuge, resuspended in 10% volume of 20 mM Tris pH 8.0, containing 300 mM NaCl and 10% Glycerol. The mixture was sonicated for 30 seconds on and off each for 6 cycles, and then centrifuged at 12000 rpm for 30 minutes at 4 °C. The supernatant fraction was passed over a Nickel NTA (BioVision, USA) column as per the manufacturer's protocol. The column was washed twice with 10 times the bed volume with 20 mM Tris pH 8.0, with 300 mM NaCl, 10% Glycerol and 20 mM Imidazole. The protein was eluted with 20mM Tris pH 8.0, 300 mM NaCl, 10% Glycerol and 300 mM Imidazole with ~ 5 times the bed volume in multiple fractions. The protein was polished over Sephacryl S-300 (GE Healthcare, USA, GE17-0599-10) following overnight dialysis at 4°C in 1X PBS and 50% Glycerol. The filter (0.25 micron) sterilized protein was stored at -20°C in working aliquots.

### Protein-protein interaction

The human proteome microarray 2.0 analysis was performed as a paid service from CDI NextGen Proteomics, MD, USA. Recombinant LCN-2 was analyzed for protein-protein interaction profiling on the HuProtTM v3.1 human proteome array and the sample was probed on array plates at 1 µg/mL, with data analyzed using GenePix software. Hit identification was assessed as the ratio of median value of the foreground to the median of the surrounding background for each protein probe on the microarray, followed by normalization to the median value of all neighboring probes within the 9x9x9 window size and represented as the significance of the probe binding signal difference from random noise (Z-Score). The cutoff Z-score was 6 in this study for the triplicate analysis; only protein interactions with a Z-score above 6 were considered.

### Enzyme-linked immunosorbent assay (ELISA)

The RPE choroid complexes harvested from freshly enucleated mouse eyes were kept on ice and then homogenized in 300 µL of complete extraction buffer (Abcam, USA, Cat# ab193970). The homogenized tissue was used to perform ELISA on 96-well microtiter plates coated with tissue lysates and incubated overnight at 4 °C. The plates were blocked with 5% BSA for 2 h. After washing, 50 µl of appropriate primary antibody, diluted to 1:1000 was added to each well and incubated for 2 h at room temperature. Bound cytokine was detected with secondary IgG peroxidase (Sigma Aldrich, USA). The color was developed with TMB substrate solution (BD Pharmingen, USA). The reaction was stopped with 2 N H₂SO₄ solution and absorbance was measured at 450 nm using a microplate reader.

### Clearing and imaging of whole eyes

Whole mouse eyes harvested from animals that had been injected with red CMTPX labeled neutrophils were fixed overnight in 4% paraformaldehyde. As described previously (Waxman et al., Invest. Ophthalmol. Vis. Sci., 59: 2371-2380 (2018) and Dodt et al., Nat. Methods, 4: 331-336 (2007)), eyes were subject to clearing by BA:BB through a series of PBS:Ethanol gradients to dehydrate the organs prior to clearing with a 1:2 mixture of benzyl alcohol (Sigma, 305197) and benzyl benzoate (Sigma, B6630). After the samples were visibly clear, they were mounted in BA:BB solution between cover glass.

Each eye was scanned using the RS-G4 ribbon scanning confocal (Caliber ID) fitted with a 20x/1.00 Glyc (CFI90 20XC, Nikon), correction collar set to 1.50. Linear interpolation of 561 nm laser excitation (iChrome-MLE-LFA, Toptica) was set between 15-30% power, top to bottom of z-stack. Emission was detected using a 630/69 band-pass filter, PMT settings were HV, 85; offset, 5. Voxels measured (0.395 × 0.395 × 5.33µm). Each sample required approximately 3.5 hours of total acquisition time. Imagery was collected at 16bit pixel depth and comprised approximately 65GB per eye. Images were collected as ribbons and were stitched and assembled using custom algorithms in MATLAB v2017b. Each dataset was converted to Nikon ND2 format and deconvolved with a custom NIS-Elements application configured for the Richardson-Lucy algorithm, line-scanning confocal, image noise level high and 0.76 µm pinhole. The deconvolved images were then converted to IMS format and loaded in to Imaris 9.2.1 (Bitplane). Prior to any analysis in Imaris, a Gaussian Filter was applied with a filter width of 0.395.

Neutrophils were quantified using the spot count function in Imaris Surpass. Spots were quantified over the entire image and then manually edited to maintain only those spots that were within the retina and Schlemm's canal. Finally, all remaining spots were filtered by volume to eliminate any structure that did not fall between 728-5800 µm³ (11-22 µm diameter), an approximate diameter of neutrophils, and to eliminate structures that were falsely selected during spot counting. Imaris surpass spot counting parameters were the same for all datasets: Enable Region Growing = true; Estimated Diameter = 10.0 µm; Background Subtraction = true; "Quality" above 190; Region Growing Type = Local Contrast; Region Growing Manual Threshold = 139.744; Region Growing Diameter = Diameter From Volume.

### RNAseq analysis

RPE-Choroid from enucleated eyes harvested from 5 and 10 month old *Cryba1*^{fl/fl} and *Cryba1* cKO mice (n=4), respectively, were subjected to total RNA isolation as previously described (Broggi et al., Nat. Immunol., 18: 1084-1093 (2017)). Approximately 30 ng/µL total RNA was used to perform RNA-sequencing as a paid service from DNA Link, USA. All sequence reads were mapped to the reference genome (NCBI37/mm⁹) using the RNAseq mapping algorithm included in CLC Genomics Workbench. The maximum number of mismatches allowed for the mapping was set at 2. To estimate gene expression levels and analyze for differentially expressed genes among the different groups, RPKM was calculated.

### Co-Immunoprecipitation

To evaluate the association between LCN-2/Dab2 and also Dab2/integrin β1 in different experimental conditions, cultured neutrophils from different experimental groups were subjected to co-immunoprecipitation (Co-IP) using the Pierce^{™} Co-Immunoprecipitation Kit (Thermo Fisher, USA, Cat# 26149). The cells were sonicated in IP Lysis/Wash Buffer (provided in the kit) plus 1% protease inhibitors (Sigma Aldrich, USA). The total lysates were processed with the kit according to the instructions. Seventy micrograms of lysates of each group were immunoprecipitated with 10 µg immobilized LCN-2 and dab2 antibodies respectively at 4 °C overnight. Normal rabbit IgG (Santa Cruz, USA) was used as the negative control. After elution, samples were loaded (15 µg per well) in SDS-PAGE and western blot was performed.

### Sub-retinal injection of neutrophils in NOD-SCID mice and OCT

NOD-SCID mice (NOD.CB17-Prkdescid/J, Jackson Laboratories, USA, male, 4-5 weeks old) were used for the study. A large sample size, n=10, was taken to nullify any experimental anomaly. Mice were anaesthetized and sub-retinal injections of neutrophils from different experimental groups or recombinant LCN-2 protein were given as described previously (Maruotti et al., Proc. Natl. Acad. Sci. U.S.A., 112: 10950-10955 (2015)). Seven days after treatment, the NOD-SCID mice were anaesthetized by intraperitoneal injection of a ketamine and xylazine mixture and then subjected to Fundus imaging along with OCT analysis using the Bioptigen Envisu R2210 system. OCT images were analyzed on optical sections (100 sections per retina) from each eye ranging from -2.0 to +2.0 mm with respect to the optic nerve head (ONH) using the FIJI-ImageJ (NIH) plugin provided with the instrument along with Diver 2.4 software (Bioptigen). After the experiment, the animals were euthanized with CO₂ gas and the eyes were harvested for further experiments.

### Hematoxylin-Eosin staining

Eyes from NOD-SCID mice were fixed in 2.5% glutaraldehyde followed by formalin, transferred to graded ethanol and dehydrated followed by embeding in methyl methacrylate. Sections of 1 µm were cut and stained with hematoxylin and eosin and observed under a light microscope.

### Statistics and Reproducibility

Statistical analysis was performed with Microsoft Excel and GraphPad Prism 6 software for Windows, using one-way ANOVA. Group means were compared using Tukey's post hoc test, with significance being set at *P*< 0.05. For experiments with human samples, comparisons between control and AMD groups were performed by Mann-Whitney test with significance being set at p < 0.05, the data distribution was determined by the Shapiro-Wilk normality test. Center lines and edge lines in box plot indicate medians and interquartile range, respectively and whiskers indicate the most extreme data points. The analyses were performed on triplicate technical replicates. Results are presented as mean ± standard deviation (SD).

### EXAMPLE 2

This example demonstrates infiltration of neutrophils in AMD and in a mouse model.

As in human AMD, *Cryba1* cKO mice present with immune cell infiltration into the retina with aging. Flow cytometry analysis for the entire retinal cell population from posterior eyecups was performed by gating for CD45^{high}CD11b⁺ cells (monocytes, macrophages, and neutrophils). The relative number of neutrophils (cells positive for Ly6C^{high}Ly6G⁺) among CD45^{high}CD11b⁺ cells in the tissue was determined, by simultaneously labelling cells with appropriate antibodies as previously described (Wu et al., Nat. Commun., 9: 4777 (2018)). While not increased in 2 month old *Cryba1* cKO retina, by 4 months, when an AMD-like phenotype is apparent in this mouse model, CD45^{high}CD11b⁺Ly6C^{high}Ly6G⁺ neutrophils were increased nearly 3-fold relative to *Cryba1*^{fl/fl} control retinas, and continued to increase with age, as seen in the 13 month old *Cryba1* cKO retina with respect to aged control mice. Furthermore, immunofluorescent analysis of retinal flatmounts from *Cryba1* cKO mice confirmed an elevated number of Ly6G⁺ cells in the retina relative to age-matched controls. An increase in sub-retinal neutrophils, as determined by Ly6G⁺ staining of RPE flatmounts, was also observed in *Cryba1* cKO mice relative to age-matched controls.

The percentage of neutrophils and their activation status in human early, dry AMD was studied by phenotyping the cells in peripheral blood by flow cytometry using appropriate gating strategies. An increase in the proportion of CD66b⁺ neutrophils within the total CD45⁺ (leukocyte) population was observed in peripheral blood of AMD patients compared to control subjects. Further, an increased number of activated neutrophils (CD45⁺CD66b^{high}) was observed in peripheral blood with no change in the number of inactive neutrophils (CD45⁺CD66b^{low}). A substantial increase in the total number of IFNλ. receptor (IL-28R1)-positive leukocytes (CD45⁺IL-28R1⁺) in the peripheral blood of AMD patients also was observed. Moreover, IL-28R1⁺ activated neutrophils (CD66b^{high}) were a markedly higher proportion of total neutrophils (CD66b⁺ cells) in peripheral blood from AMD subjects compared to age-matched controls. Immunolocalization studies show presence of CD66b⁺ neutrophils in human tissue sections from normal and AMD samples.

An increased number of neutrophils are present in the retina of human AMD patients compared to aged-matched control subjects (Gosh et al., J. Pathol., 241: 583-588 (2017)). However, IL28R1⁺ expression is evident on CD66b⁺ neutrophils only in retinal sections of AMD patients, but not in controls, indicating that activated neutrophils home into the retina of only early AMD patients.

These results indicate a greater propensity for IL-28R1⁺ activated neutrophils to home into the eye, giving a probable scenario for the role of IFNA-mediated signaling in these infiltrating neutrophils. Once in the area of inflammation, neutrophils release Neutrophil Extracellular Traps (NETs), which can damage host tissue in immune-mediated diseases. Indeed, early, dry AMD eyes showed increased staining for the NET markers, myeloperoxidase (MPO), neutrophil elastase and citrullinated histone H3 as compared to age-matched control eyes.

Taken together, these results support the idea that there is increased neutrophil infiltration into the retina during early, dry AMD.

### EXAMPLE 3

The example identifies factors promoting neutrophil infiltration into the retina.

RNAseq analysis was performed on retinal tissue obtained from 5 and 10 month old *Cryba1* cKO and floxed control mice in order to identify soluble factors, including cytokines and chemokines released from the retina, that may promote neutrophil infiltration. Activation of genes related to cGAS/STING pathway was observed (Fig. 1). Additionally, a major increase in the levels of IFNs, including IFNα, IFNγ and IFNλ, as well as CXCL1 and CXCL9, was observed in the aged *Cryba1* cKO retinas compared to control (Fig. 2A). ELISA was performed to further confirm these results (Fig. 2B).

To identify which cell types express IFNλ in the retina, immunofluorescence studies were conducted. Elevated levels of IFNλ. were observed in RPE cells of human dry AMD patients compared to age-matched controls. Western analysis confirmed increased IFNλ and CXCL1 protein in human AMD RPE/choroid lysates, compared to control. In addition, an increase in the levels of IFNα and IFNλ1 in the plasma and AH of early AMD patients was observe compared to controls, but levels of IFNλ2/3 were not different in AMD patients compared to controls. The plasma levels of IFNγ showed substantial increase in AMD patients compared to control, but no such change was found in the AH. IFNβ and VEGF levels in the plasma and AH of AMD patients were not different from controls.

Thus, these results support a pro-inflammatory milieu in the eye, with a probable involvement of IFNλ, which is secreted from the diseased RPE thereby eliciting an inflammatory response. Therefore, the inventors hypothesized that the increased levels of IFNλ might be the key factor that promotes the neutrophil activation and infiltration into the retina, since IFNλ receptor (IL28R1) is expressed on circulating neutrophils.

In addition to soluble factors, neutrophils also require adhesion molecules for their transmigration into the site of injury. Neutrophils adhere to endothelial cells when their integrins interact with endothelial cell immunoglobulin superfamily members, such as ICAM-1 and VCAM-1 (two important adhesion molecules on endothelial cells), which enables them to transmigrate into diseased or injured tissue. Elevated levels of ICAM-1 as well as VCAM-1 were obsereved in the retina of aged *Cryba1* cKO mice and human early, dry AMD patients respectively, relative to age-matched controls.

### EXAMPLE 4

This example demonstrates that IFNλ triggers LCN-2 expression and neutrophil activation.

It has been previously reported that IFNλ triggers phosphorylation and nuclear translocation (activation) of STAT1 (Maher et al., Cancer Bio. Ther., 7, 1109-1115 (2008)). During early AMD, STAT1 activation is critical for *lcn2* gene expression (Ghosh et al., J. Pathol., 241, 583-588 (2017)). LCN-2 is an adipokine, known to be important for neutrophil activation and innate immune function. In fact, binding of NFκB and STAT1 to the promoter of LCN-2 causes pathogenicity (Ghosh et al., *supra,* and Zhao et al., Mol. Metab., 2: 161-170 (2013)).

Mouse bone marrow-derived neutrophils cultured with either recombinant IFNλ or with conditioned medium from primary cultured RPE cells overexpressing IFNλ to simulate the increased IFNλ levels observed in the RPE of human AMD patients, exhibit increased levels of LCN-2 and phosphorylated STAT1. Moreover, IFNλ-exposed neutrophils showed a major increase in reactive oxygen species (ROS) levels and phagocytosis. Increased formation of NETs was evident because of the prevalence of extracellular nuclear material (stained with DAPI), which showed increased staining for myeloperoxidase (MPO) and citrullinated Histone H3, known markers of NETs.

Thus, the data supports that IFNλ not only induces STAT1-mediated LCN-2 expression, but also potentiates neutrophil activation.

### EXAMPLE 5

This example demonstrates that LCN-2 activated neutrophils cause retinal degeneration
Ribbon-scanning confocal microscopy (RSCM) was applied as a means to rapidly image red CMTPX-tagged neutrophils within an entire NOD-SCID immune-deficient mouse eye to validate transmigration of activated neutrophils. The mice were intravenously injected with bone marrow-derived wild type (WT) neutrophils, bone marrow-derived neutrophils from LCN-2^{-/-} (knockout) mice, WT neutrophils treated with IFNλ, or IFNλ treated neutrophils from LCN-2^{-/-} mice. To demonstrate homing of activated neutrophils to specific regions of the eye, RSCM paired with benzyl alcohol benzyl benzoate (BABB) clearing of NOD-SCID mouse eyes was performed. The clearing procedure makes the refractive index consistent throughout the eye, thereby making the tissue transparent and allowing image acquisition throughout the depth of the whole organ.

NOD-SCID mice administered with red CMTX-tagged WT neutrophils showed little infiltration into the eye and similarly, not many neutrophils derived from LCN-2^{-/-} mice infiltrated the eye. The data clearly suggest that neutrophils home mostly into the choroid in both of these conditions, but due to the lack of stimuli from IFNλ in WT neutrophils and probably due to the perturbed migratory signaling axis in the LCN-2^{-/-} mice, these cells fail to cross the intra-ocular compartments in considerable numbers through the blood-retinal or blood-aqueous barrier.

Interestingly, red CMTPX-tagged neutrophils treated with IFNλ showed a noticeable number of neutrophils infiltrating the eye, mostly into the retina relative to control (Fig. 3). During early stages of AMD, neutrophils migrate from the peripheral blood into the intra-ocular compartments in response to a chemotactic cue, which was identified as IFNλ. In addition, NOD-SCID mice injected with IFNλ-treated LCN-2^{-/-} neutrophils showed very few infiltrating cells into the retina compared to mice injected with untreated LCN-2^{-/-} neutrophils demonstrating that neutrophil infiltration into the eye from the peripheral circulation is likely due to the IFNλ triggered LCN-2 activation.

To further validate the observations that increased LCN-2 levels induced by IFNλ in the transmigrating neutrophils can potentiate outer retinal degeneration, NOD-SCID mice were injected with bone marrow-derived WT neutrophils, bone marrow-derived neutrophils from LCN-2^{-/-} mice, WT neutrophils treated with IFNλ, neutrophils treated with conditioned medium from primary cultures of RPE cells overexpressing IFNλ, or with recombinant LCN-2.

After 7 days, Optical Coherence Tomography (OCT) analysis showed that mice injected with either IFNλ-treated WT neutrophils or recombinant LCN-2 exhibited alterations in the RPE and photoreceptor (inner and outer segments) layers (Figs. 4(iii)-(v)). Quantitative analysis by spider plot revealed decreased thickness of these layers in the experimental groups (Fig. 4(ix)). No noticeable changes were observed in mice treated with vehicle and/or WT neutrophils (Figs. 4(i), (ii), and (ix)). In addition, LCN-2^{-/-} neutrophils, as well as LCN-2^{-/-} neutrophils treated with IFNλ, showed no degenerative changes (Figs. 4(vi)-(ix)), suggesting a pathogenic role of LCN-2 in retinal degeneration.

Hematoxylin-eosin staining of retinal sections from NOD-SCID mice, injected with either IFNλ-treated WT neutrophils or recombinant LCN-2, showed degenerative changes in the outer nuclear layer (ONL) along with photoreceptor layer (disruption of the inner and outer segments [IS/OS] junction) and RPE-Bruch's membrane-choriocapillaris complex, relative to vehicle control or WT neutrophil injected mice. Further, a thickness measurement of the retinal layers from these sections by spider plot showed severe loss or thinning of IS/OS and RPE layers in mice injected with either IFNλ-treated WT neutrophils or recombinant LCN-2, relative to vehicle or WT neutrophil-treated groups. In addition, immunofluorescence studies confirm increased photoreceptor and RPE cell loss in these mice, as evident from reduced staining for rhodopsin (labels rod photoreceptors) and RPE 65 (retinal pigment epithelium-specific 65 kDa protein) in the retina of NOD-SCID mice, injected with either IFNλ-treated WT neutrophils or recombinant LCN-2, with respect to controls.

This NOD-SCID mouse data provides evidence that IFNλ triggers LCN-2 activation in neutrophils, thereby inducing transmigration into the retina and potentiating retinal degeneration. As such, administering one or more inhibitors of IFNλ would treat or prevent retinal degeneration to rescue an AMD-like phenotype.

### EXAMPLE 6

This example demonstrates that association of LCN-2/Dab2 regulates neutrophil infiltration.

The above-described observations prompted the further investigation of the possible molecular mechanisms by which neutrophils infiltrate into the retina and thereby contribute to the pathogenesis of AMD. It has previously been shown that LCN-2 regulates neutrophil chemotaxis and cell migration in cancer cells (Shao et al., J. Invest. Dermatol., 136, 1418-1428 (2016), and Du et al., Biochim. Biophys. Acta, 1853: 2240-2250 (2015)). To ascertain if IFNX-mediated LCN-2 activation in neutrophils contributes to the increased adhesion and transmigration, a human proteome high-throughput array was performed to identify LCN-2 binding partners that may play a specific role in cell adhesion and migration. LCN-2 was determined to interact with Dab2. This was confirmed by a pull-down assay, which showed an increased association between LCN-2 and Dab2 in IFNλ-exposed neutrophils as compared to untreated or control conditioned media treated neutrophils.

It has previously been reported that, Dab2 binds to integrin β1 and regulates its internalization, thereby modulating cell migration. It is also known that Dab2 is a negative regulator of cell adhesion particularly during inflammation. Moreover, extracellular integrin β1 expression drives cell adhesion on the endothelial cell surface in various tissues thereby facilitating transmigration into the tissue. The inventors hypothesized that this increased association between LCN-2 and Dab2 may regulate extracellular integrin β1 level by modulating the Dab2/integrin β1 axis, thereby promoting neutrophil adhesion and transmigration into the retina.

To explore the role of LCN-2, bone marrow-derived neutrophils from WT and LCN-2^{-/-} mice that were cultured with either recombinant IFNλ. or conditioned medium from IFNλ overexpressing RPE cells were used. Flow cytometry studies revealed an increase in extracellular integrin β1 expression in IFNλ-exposed neutrophils from wild type mice concomitant with decreased of integrin β1. In addition, co-immunoprecipitation data did not show any change in the binding between Dab2 and integrin β1 upon IFNα, exposure with respect to controls.

These results support an alteration in the Dab2-mediated cellular internalization of integrin β1 in the IFNλ-exposed neutrophils, particularly due to the increased association between LCN-2 and Dab2 in the IFNλ-exposed cells.

Since neutrophils were observed homing into the eye in NOD-SCID mice that were injected with IFNλ-exposed LCN-2^{-/-} neutrophils, it is likely that the expression of adhesion-associated surface proteins is downregulated in the absence of LCN-2. Based on these observations, the inventors postulated that LCN-2 regulates the expression of extracellular adhesion molecules, which in turn modulates cell adhesion and transmigration. However, there could be involvement of putative redundant pathways in regulating neutrophil infiltration upon exposure to IFNλ.

Intensified neutrophil adhesion on fibrinogen-coated plates and transmigration of IFNλ-treated normal neutrophils across fibrinogen-coated transwell chambers were observed. In addition, it was discovered that there was an increase in the extracellular expression of integrin β1 on untreated neutrophils from LCN-2^{-/-} mice. This data is in sharp contrast to our previous observation that LCN-2^{-/-} neutrophils treated with IFNλ have decreased surface expression of integrin β1.

Previous studies have shown that integrin β1 surface expression in neutrophils can be modulated by a number of independent signaling cascades during inflammation. It is therefore plausible that integrin β1 in untreated LCN-2^{-/-} neutrophils is upregulated independently of IFNλ/LCN-2/Dab2 pathway. But, the extracellular integrin β1 level and its internalization were stabilized in these LCN-2^{-/-} neutrophils, even after IFNλ treatment, relative to IFNλ-exposed WT neutrophils. However, the adhesion and transmigration properties were greatly reduced in LCN-2^{-/-} neutrophils exposed to IFNλ and in integrin β1 silenced normal neutrophils, with no change in cell viability.

These results suggest that LCN-2 regulates Dab2-mediated internalization of integrin β1, which is critical for cell adhesion and migration of IFNλ-exposed neutrophils.

### EXAMPLE 7

This example describes methods of identifying IFNλ inhibitors, which can be used to inhibit neutrophil activation and to treat or delay AMD.

### Identification of Inhibitors Using DARPins

DARPins (an acronym for designed ankyrin repeat proteins) are genetically engineered antibody mimetic proteins typically exhibiting highly specific and high-affinity target protein binding as described in Plückthun et al., Annu. Rev. Pharmacol. Toxicol., 55: 489-511 (2015), and Binz et al., J. Mol. Biol., 332: 489-503 (2003)).

The DARPin consensus sequence is
DxxGxTPLHLAAxxGHLEIVEVLLKZGADVNAx (SEQ ID NO: 7), where x= any amino acid except Cys, Pro, or Gly and Z= His, Asp or Tyr.

Reverse translation of DARPIN consensus sequence to a 99 base sequence of most likely codons results in
gatnnnnnnggcnnnaccccgctgcatctggcggcgnnnnnnggccatctggaaattgtggaagtgctgctgaaataaggcgcg gatgtgaacgcgnnn (SEQ ID NO: 8), where n= any base (either A, T, G or C) without yielding Cys, Pro, or Gly.

Novel library preparation for all the predicted sequence possibilities.

Assess binding affinity to immobilized target protein IFNL1 interferon lambda 1 (*Homo sapiens*) by ribosome display, wherein IFNL1 interferon lambda 1 [Homo sapiens (human)] of Gene ID: 282618 has the following nucleotide and translated amino acid sequences.

### Nucleotide Sequence (603 nt):

ATGGCTGCAGCTTGGACCGTGGTGCTGGTGACTTTGGTGCTAGGCTTGGCCGTGG
CAGGCCCTGTCCCCACTTCCAAGCCCACCACAACTGGGAAGGGCTGCCACATTGG
CAGGTTCAAATCTCTGTCACCACAGGAGCTAGCGAGCTTCAAGAAGGCCAGGGA
CGCCTTGGAAGAGTCACTCAAGCTGAAAAACTGGAGTTGCAGCTCTCCTGTCTTC
CCCGGGAATTGGGACCTGAGGCTTCTCCAGGTGAGGGAGCGCCCTGTGGCCTTG
GAGGCTGAGCTGGCCCTGACGCTGAAGGTCCTGGAGGCCGCTGCTGGCCCAGCC
CTGGAGGACGTCCTAGACCAGCCCCTTCACACCCTGCACCACATCCTCTCCCAGC
TCCAGGCCTGTATCCAGCCTCAGCCCACAGCAGGGCCCAGGCCCCGGGGCCGCC
TCCACCACTGGCTGCACCGGCTCCAGGAGGCCCCCAAAAAGGAGTCCGCTGGCT
GCCTGGAGGCATCTGTCACCTTCAACCTCTTCCGCCTCCTCACGCGAGACCTCAA
ATATGTGGCCGATGGGAACCTGTGTCTGAGAACGTCAACCCACCCTGAGTCCACC
TGA (SEQ ID NO: 9).

### Translated amino acid sequence (200 aa):

MAAAWTVVLVTLVLGLAVAGPVPTSKPTTTGKGCHIGRFKSLSPQELASFKKARDA
LEESLKLKNWSCSSPVFPGNWDLRLLQVRERPVALEAELALTLKVLEAAAGPALED
VLDQPLHTLHHILSQLQACIQPQPTAGPRPRGRLHHWLHRLQEAPKKESAGCLEASV
TFNLFRLLTRDLKYVADGNLCLRTSTHPEST (SEQ ID NO: 10).

The screened DARPin targeting human IFNL1 will be used as candidate drug(s) to neutralize IFNL1 expression in retina of human dry AMD patients.

### RNA interference (RNAi)

The human IFNL1 interferon lambda 1 nucleic acid sequence (SEQ ID NO: 9) was analyzed and the following shRNA target sequences were identified (Table 1).

**Table 1: IFNL1 shRNA target sequences**

| **Target sequences** | **shRNA target sequences for IFNL1 (IFN-lambda 1)** | **Corresponding residues of IFNL1 (SEQ ID NO: 9)** |
|---|---|---|
| 1 | AGGAGCTAGCGAGCTTCAAGA (SEQ ID NO: 11) | 134-154 |
| 2 | GGAGCTAGCGAGCTTCAAGAA (SEQ ID NO: 12) | 135-155 |
| 3 | GAGCTAGCGAGCTTCAAGAAG (SEQ ID NO: 13) | 136-156 |
| 4 | AGCTAGCGAGCTTCAAGAAGG (SEQ ID NO: 14) | 137-157 |
| 5 | GCCTTGGAAGAGTCACTCAAG (SEQ ID NO: 15) | 166-186 |
| 6 | AGGCATCTGTCACCTTCAACC (SEQ ID NO: 16) | 497-517 |
| 7 | GGCATCTGTCACCTTCAACCT (SEQ ID NO: 17) | 498-518 |

Each of these sequences will be cloned and packaged into AAV2 and/or AAV8 delivery system.

### EXAMPLE 8

This example demonstrates that that βA1-crystallin is required for lysosomal function in RPE cells.

*Cryba1* has two gene products: βA3- and βA1-crystallin. To study the functions of βA3- and βA1-crystallin, a βA3-crytallin knockout and a βA1-crytallin knockout were created. As shown in Fig. 5A, the wild-type sequence of *Cryba1* shows dual ATG start sites for βA3- and βA1-crystallin. In the βA1-knockdown mutant, CCACC was inserted immediately before the first start site to strengthen the Kozak consensus sequence, thereby making the first start codon stronger (Fig. 5B). In the βA3-knockout mutant, a change from an A to G change that inactivates the first start site (Fig. 5C). A silent mutation (ACC to ACG) also was introduced to prevent the binding and re-cutting of the sequence by gRNA after homology-directed repair (Fig. 5C).

Knockdown of βA1-crystallin caused inflammation in RPE cells as indicated by increased protein levels of interferons (e.g., IFNα, IFNβ, and IFNλ), CXCL1, and TNFα (Fig. 6). These increased cytokine levels were not observed in RPE cells from wild-type or βA3-crystallin knockout mice (Fig. 6).

Additionally, knockdown of βA1-crystallin caused oxidative damage in RPE cells as indicated by superoxide (SOD2) expression (Figs. 7A-B). A significant increase in SOD2 expression was observed in *Cryba1* cKO and βA1-crystallin knockdown mice relative to that in RPE cells of wild-type mice (Fig. 7B). The increased SOD2 expression was not observed in RPE cells from βA3-crystallin knockout mice (Fig. 7B).

These results demonstrate that loss of βA1-crystallin resulted in inflammation and oxidative damage in RPE cells, and support that the administration of βA1-crystallin mitigates alterations in lysosomal function in RPE cells, which alterations are observed in disorders such as AMD, diabetic retinopathy, and diabetic macular edema, and neurodegenerative diseases, such as Alzheimer's disease or Parkinson's disease.

The βA1-crystallin can be administered to RPE cells by any suitable means, including in a vector (e.g., AAV vector). An exemplary vector (scCAG_humCryba1) is shown in Fig. 8 and its corresponding nucleic acid sequence is shown in Fig. 9.

### EXAMPLE 9

This example demonstrates that βA1-crystallin can be used in the treatment of diabetic retinopathy (DR).

DR is one of the leading causes of blindness with no cure available at the present time and is estimated to afflict 400 million people in 2020. Several decades of DR research defined the disease more accurately as a neurodegenerative disease that precedes and coexist with vascular changes. One important gap in the understanding of the disease is that during the different stages of DR progression, a major player that may contribute to DR pathology, i.e. the contribution of the retinal glial population, has not been taken into account in pathologic retinal modeling.

In recent years, inhibitors for protein tyrosine phosphatase 1B (PTP1B), a phosphatase that is ubiquitously expressed and a known negative regulator of leptin and insulin signaling pathways, have been in clinical trials for the treatment of type 2 diabetes. Increased PTP1B phosphatase activity has been implicated in several disease processes, including DR. The majority of these small molecules are pharmacological inhibitors and therefore have side effects and also lack the specificity that is absolutely essential for targeting PTP1B. Therefore, there is a desire for new inhibitors of PTP1B.

The inventors discovered a protein (biological inhibitor) that binds directly to PTP1B to inhibit the abnormal activity of PTP1B in DR and showed the efficacy of the drug in a unique rat model of the disease (Nuc1).

The Nuc1 rat model is characterized by a spontaneous mutation in *Crybal* gene that encodes βA3/A1-crystallin protein. Heterozygotes show nuclear cataracts, and homozygotes show microphthalmia. The Nuc1 rat model shows symptoms of persistent fetal vasculature (PFV) disease of the eye. The Nuc1 rat model for DR is unique since it is the only animal model that shows microaneurysms as the disease progresses, a hallmark of DR in human patients (see Figs. 11 and 12).

In a human proteome high-throughput array (CDI Laboratories, Inc.), βA3/A1-crystallin was determined to interact with PTP1B, an enzyme that is the link between metabolism and inflammation. In a pull-down assay, βA3/A1-crystallin bound to PTP1B. PTP1B activity is regulated by βA3/A1-crystallin, two closely related polypeptides produced by two different translational start sites that have an important regulatory function in astrocytes. βA3/A1-crystallin was determined to be an uncompetitive inhibitor of PTP1B (see Fig. 13). Co-immunoprecipitation assays demonstrated that PTP1B was a binding partner of βA3/A1-crystallin protein in wild-type astrocytes but this binding was lost in the βA3/A1-crystallin mutant Nuc1 cells.

The inventors postulated that high glucose levels in diabetes trigger glial abnormalities (particularly in astrocytes) that affect the homeostasis of the retina.

Loss of βA3/A1-crystallin protein in the Nuc1 rat model showed increased PTP1B activity in astrocytes, which was significantly upregulated when astrocytes from Nuc1 rats were treated with high glucose (HG) (see Fig. 14A). Elevated levels of PTP1B also were observed in the vitreous humor of human DR patients (see Fig. 14B). Lower levels of crystallin compared to PTP1B were observed in samples from the vitreous humor of human patients with progressive DR (PDR), but this was not observed in normal samples (control) (Figs. 14C-14D).

A βA1-crystallin construct for gene therapy was prepared according to Figs. 15A-15C. PTP1B activity was rescued to normal wild type levels when □A1-crystallin was overexpressed in astrocytes from Nuc1 rats following administration of the βA1-crystallin vector (see Fig. 16).

These results supported the homology modeling data that the binding of the open conformer of βA1 to PTP1B blocks the substrate access to the active site and thereby inhibits the tyrosine-phosphatase activity of PTP1B, which is necessary for maintaining the homeostasis of PTP1B activity in astrocytes. Additionally, administration of the βA1-crystallin vector rescued the DR phenotype in Nuc1 rats (see Fig. 17).

These observations prompted the inventors to delineate the extracellular flux in astrocytes using the seahorse XF platform by selectively blocking glycolysis and OxPhos (oxidative phosphorylation) pathways with 2-Deoxyglucose and Rotenone/Antimycin, respectively. An increase in the extracellular acidification rate (ECAR - an indirect measurement of glycolysis) and a decrease in mitochondrial respiration (oxygen consumption rate or OCR) were observed in Nuc1 astrocytes treated with high glucose compared to wild-type cells exposed to high glucose (see Figs. 18A-18B). With overexpression of βA1-crystallin in Nuc1 astrocytes, ECAR was decreased and OCR was increased in Nuc1 astrocytes to wild-type levels (see Figs. 18A-18B). Moreover, by inhibiting PTPB1 expression by shRNA, ECAR was decreased and OCR increased in Nuc1 astrocytes.

In accordance with the increase in ECAR levels in high glucose-treated Nuc1 astrocytes, a significant increase in lactate levels in these cells also was observed, indicative of increased glycolytic flux (see Fig. 19A). This was rescued upon βA1-crystallin overexpression (see Fig. 19A) or PTP1B knockdown. Taken together, the data confirms PTP1B and βA1-crystallin interaction is essential for maintaining normal glycolysis and oxidative phosphorylation in astrocytes.

It is known that OxPhos is key for normal neuroprotective function rendered by astrocytes and that decrease in OxPhos results in the mitochondria not making enough ATP leading to the generation of excess mitochondrial ROS (mROS). Nuc1 astrocytes exposed to high glucose show a significant increase in mROS, relative to WT cells (see Fig. 19B), which could be rescued either by βA1-crystallin overexpression (see Fig. 19B) or PTP1B knockdown.

In a multifactorial disease like DR, a group of patients with high glucose levels in diabetes might trigger an abnormal stoichiometry between PTP1B and βA1-crystallin in astrocytes-abnormalities that might link the metabolic changes to immune dysregulation in the retina, which can possibly lead to the breakdown of the blood-retinal barrier (BRB) with consequent infiltration of immune cells and lead to severity of the disease. This can be efficiently blocked using open conformer of βA1-crystallin that shows the inhibitory effect on the tyrosine-phosphatase activity of PTP1B.

Using the gene therapy approach to overexpress βA1-crystallin to rescue the DR-like phenotype in the Nuc1 rat model and electroretinography (ERG), it was observed that gene therapy of βA1-crystallin could successfully improve retinal function. Therefore, βA1-crystallin is a first of its kind inhibitor that can be used as a therapy for DR.

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

Although the present invention is defined in the attached claims, it should be understood that the present invention can also (alternatively) be defined in accordance with the following embodiments:
1. A method of inhibiting neutrophil activation in retinal pigment epithelial (RPE) cells comprising administering an inhibitor of interferon (IFN) λ to a subject in need thereof.
2. The method of embodiment 1, wherein the inhibitor is selected from the group consisting of a neutralizing anti-IFNλ antibody, an antisense oligonucleotide, a small interfering ribonucleic acid (siRNA), a small hairpin RNA (shRNA), a non-antibody binding polypeptide, or a small molecule chemical compound.
3. The method of embodiment 2, wherein the antibody is selected from the group consisting of an immunoglobulin, an antibody fragment, and an antibody analogue.
4. The method of embodiment 3, wherein the antibody is the antibody fragment selected from the group consisting of Fab, Fab', F(ab')2, F(ab'), F(ab), Fv, and scFv.
5. The method of embodiment 3, wherein the antibody is the antibody analogue selected from the group consisting of affibody, repebody, affilin, DARPin, tetranectin, microbody, peptide aptamer, and avimer.
6. The method of embodiment 2, wherein the shRNA targets one or more of the nucleotide sequences consisting of the group consisting of SEQ ID NOs: 11-17.
7. The method of embodiment 6, wherein the shRNA is comprised in a vector.
8. The method of embodiment 7, wherein the vector is an adeno-associated (AAV) vector.
9. The method of embodiment 8, wherein the AAV vector is AAV2 or AAV8.
10. The method of embodiment 1, wherein the inhibitor is administered by subretinal injection, intravitreal injection, or topical administration.
11. A method of restoring lysosomal function of RPE cells comprising administering a polypeptide comprising βA1-crystallin or a nucleic acid encoding the polypeptide to a subject in need thereof.
12. The method of embodiment 11, wherein the nucleic acid further comprises a hVMD2 promoter.
13. The method of embodiment 11, wherein the nucleic acid is comprised in a vector.
14. The method of embodiment 13, wherein the vector is an adeno-associated viral (AAV) vector.
15. The method of embodiment 14, wherein the AAV vector is AAV2 or AAV8.
16. The method of embodiment 11, wherein the polypeptide or nucleic acid encoding the polypeptide is administered by subretinal injection, intravitreal injection, or topical administration.
17. A method of rejuvenating Vacuolar-type H⁺-ATPase (V-ATPase) activity in RPE cells by modulating assembly and disassembly of the V-ATPase in a subject in need thereof.
18. The method of embodiment 1, wherein the subject has age-related macular degeneration or is at risk for developing AMD.
19. The method of embodiment 18, wherein the AMD is atrophic AMD, optionally wherein the atrophic AMD includes geographic atrophy.
20. The method of embodiment 18, wherein the AMD is wet AMD.
21. The method of embodiment 1, wherein the subject has a neurodegenerative disease or is at risk for developing a neurodegenerative disease.
22. The method of embodiment 21, wherein the neurodegenerative disease is Alzheimer's disease or Parkinson's disease.
23. The method of embodiment 1, wherein the subject has diabetic retinopathy (DR) or is at risk for developing DR.
24. A method of treating diabetic retinopathy in a subject comprising administering a polypeptide comprising βA1-crystallin or a nucleic acid encoding the polypeptide to the subject.
25. The method of embodiment 24, wherein the nucleic acid further comprises a hVMD2 promoter.
26. The method of embodiment 24, wherein the nucleic acid is comprised in a vector.
27. The method of embodiment 26, wherein the vector is an adeno-associated viral (AAV) vector.
28. The method of embodiment 27, wherein the AAV vector is AAV2 or AAV8.
29. The method of embodiment 24, wherein the polypeptide or nucleic acid encoding the polypeptide is administered by subretinal injection, intravitreal injection, or topical administration.
30. The method of embodiment 1, wherein the subject is a human.

## Claims

1. A polypeptide comprising βA1-crystallin or a nucleic acid encoding the polypeptide for use in a method of restoring lysosomal function of RPE cells, the method comprising administering said polypeptide or said nucleic acid to a subject in need thereof.

2. The polypeptide or the nucleic acid for use of claim 1, wherein the nucleic acid further comprises a hVMD2 promoter.

3. The polypeptide or the nucleic acid for use of claim 1 or claim 2, wherein the nucleic acid is comprised in a vector.

4. The polypeptide or the nucleic acid for use of claim 3, wherein the vector is an adeno-associated viral (AAV) vector.

5. The polypeptide or the nucleic acid for use of claim 4, wherein the AAV vector is AAV2 or AAV8.

6. The polypeptide or the nucleic acid for use of any one of claims 1-5, wherein the polypeptide or nucleic acid encoding the polypeptide is administered by subretinal injection, intravitreal injection, or topical administration.

7. A polypeptide comprising βA1-crystallin or a nucleic acid encoding the polypeptide for use in a method of treating diabetic retinopathy in a subject, the method comprising administering said polypeptide or said nucleic acid to the subject.

8. The polypeptide or the nucleic acid for use of claim 7, wherein the nucleic acid further comprises a hVMD2 promoter.

9. The polypeptide or the nucleic acid for use of claim 7 or claim 8, wherein the nucleic acid is comprised in a vector.

10. The polypeptide or the nucleic acid for use of claim 9, wherein the vector is an adeno-associated viral (AAV) vector.

11. The polypeptide or the nucleic acid for use of claim 10, wherein the AAV vector is AAV2 or AAV8.

12. The polypeptide or the nucleic acid for use of any one of claims 7-11, wherein the polypeptide or nucleic acid encoding the polypeptide is administered by subretinal injection, intravitreal injection, or topical administration.

13. The polypeptide or the nucleic acid for use of any one of claims 1-12, wherein the subject is a human.
